(19) Europäisches Patentamt
European Patent Office
Office européen des brevets

(11) EP 2 560 987 B1

(12) **EUROPÄISCHE PATENTSCHRIFT**

(45) Veröffentlichungstag und Bekanntmachung des Hinweises auf die Patenterteilung:
**20.02.2019 Patentblatt 2019/08**

(21) Anmeldenummer: **11713717.4**

(22) Anmeldetag: **15.03.2011**

(51) Int Cl.:
*C07K 14/21* (2006.01) *C12P 7/00* (2006.01)
*C12P 7/04* (2006.01) *C12N 15/09* (2006.01)

(86) Internationale Anmeldenummer:
**PCT/EP2011/053834**

(87) Internationale Veröffentlichungsnummer:
**WO 2011/131420 (27.10.2011 Gazette 2011/43)**

(54) **BIOKATALYTISCHES OXIDATIONSVERFAHREN MIT ALKL-GENPRODUKT**

BIOCATALYTICAL OXIDATION PROCESS WITH ALKL GENE PRODUCT

PROCÉDÉ D'OXYDATION PAR BIOCATALYSE A L'AIDE DU PRODUIT GENIQUE ALKL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.04.2010 DE 102010015807**

(43) Veröffentlichungstag der Anmeldung:
**27.02.2013 Patentblatt 2013/09**

(73) Patentinhaber: **Evonik Degussa GmbH**
**45128 Essen (DE)**

(72) Erfinder:
- **PÖTTER, Markus**
**48149 Münster (DE)**
- **SCHMID, Andreas**
**44229 Dortmund (DE)**
- **BÜHLER, Bruno**
**44143 Dortmund (DE)**
- **HENNEMANN, Hans-Georg**
**45770 Marl (DE)**
- **JULSING, Mattijs Kamiel**
**44137 Dortmund (DE)**
- **SCHAFFER, Steffen**
**45699 Herten (DE)**
- **HAAS, Thomas**
**48161 Münster (DE)**
- **SCHREWE, Manfred**
**44135 Dortmund (DE)**
- **CORNELISSEN, Sjef**
**2200 Kopenhagen N (DK)**
- **ROOS, Martin**
**45721 Haltern am See (DE)**
- **HÄGER, Harald**
**59348 Lüdinghausen (DE)**

(56) Entgegenhaltungen:
**EP-A1- 0 277 674 EP-A1- 0 502 524**
**WO-A1-02/22845 WO-A1-2009/077461**

- **ROTHEN S A ET AL: "Biotransformation of octane by E. coli HB101(pGEc47) on defined medium: Octanoate production and product inhibition", BIOTECHNOLOGY AND BIOENGINEERING, Bd. 58, Nr. 4, 20. Mai 1998 (1998-05-20), Seiten 356-365, XP002645598, ISSN: 0006-3592 in der Anmeldung erwähnt**
- **FAVRE-BULLE O ET AL: "Bioconversion of n-octane to octanoic acid by a recombinant Escherichia coli cultured in a two-liquid phase bioreactor.", BIO/TECHNOLOGY (NATURE PUBLISHING COMPANY) APR 1991 LNKD- PUBMED:1367010, Bd. 9, Nr. 4, April 1991 (1991-04), Seiten 367-371, XP001525869, ISSN: 0733-222X in der Anmeldung erwähnt**
- **SCHNEIDER SILKE ET AL: "Biocatalyst engineering by assembly of fatty acid transport and oxidation activities for in vivo application of cytochrome P-450BM-3 monooxygenase", APPLIED AND ENVIRONMENTAL MICROBIOLOGY, Bd. 64, Nr. 10, Oktober 1998 (1998-10), Seiten 3784-3790, XP002645599, ISSN: 0099-2240 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- CHEN QI ET AL: "Physiological changes and alk gene instability in Pseudomonas oleovorans during induction and expression of alk genes", JOURNAL OF BACTERIOLOGY, Bd. 178, Nr. 18, 1996, Seiten 5508-5512, XP002645600, ISSN: 0021-9193
- NEHER TRACY M ET AL: "Pseudomonas fluorescens ompW: plasmid localization and requirement for naphthalene uptake", CANADIAN JOURNAL OF MICROBIOLOGY, Bd. 55, Nr. 5, Mai 2009 (2009-05), Seiten 553-563, XP009149676, ISSN: 0008-4166
- BEILEN VAN J B ET AL: "DNA SEQUENCE DETERMINATION AND FUNCTIONAL CHARACTERIZATION OF THE OCT-PLASMID-ENCODED ALKJKL GENES OF PSEUDOMONAS OLEOVORANS", MOLECULAR MICROBIOLOGY, WILEY-BLACKWELL PUBLISHING LTD, GB, Bd. 6, Nr. 21, 1. Januar 1992 (1992-01-01) , Seiten 3121-3136, XP001098364, ISSN: 0950-382X, DOI: DOI:10.1111/J.1365-2958.1992.TB01769.X
- VAN BEILEN JAN B ET AL: "Analysis of Pseudomonas putida alkane-degradation gene clusters and flanking insertion sequences: Evolution and regulation of the alk genes", MICROBIOLOGY (READING), Bd. 147, Nr. 6, Juni 2001 (2001-06), Seiten 1621-1630, XP002645601, ISSN: 1350-0872
- BEILEN VAN J B ET AL: "GENETICS OF ALKANE OXIDATION BY PSEUDOMONAS OLEOVORANS", BIODEGRADATION, KLUWER ACADEMIC PUBLISHERS, NL, Bd. 5, Nr. 3/04, 1. Dezember 1994 (1994-12-01), Seiten 161-174, XP001001243, ISSN: 0923-9820, DOI: DOI:10.1007/BF00696457

## Beschreibung

Gebiet der Erfindung

Gegenstand der Erfindung ist ein biokatalytisches Verfahren zur Oxidation von organischen Verbindungen unter Zuhilfenahme eines alkL-Genproduktes sowie in diesem Verfahren eingesetzte Mikroorganismen.

Stand der Technik

Beispielsweise das OCT-Plasmid von *Pseudomonas putida* enthält ein alkL-Gen. Dieses Plasmid kodiert des Weiteren für Genprodukte, die für die Alkan-Degradation verantwortlich sind. Diese Alkan-Degradationsgene sind auf dem *Pseudomonas* OCT-Plasmid in zwei alk-Operons angeordnet; das erste kodiert für die Genprodukte AlkB, AlkF, AlkG, AlkH, AlkJ, AlkK, und AlkL, das zweite für AlkS und AlkT, wobei AlkS eine regulatorische Funktion auf die Expression des ersten alk-Operons ausübt. Für eine detaillierte Übersicht und die Funktion weiterer Gene dieser alk-Operons siehe Chen et al., J Bacteriol. 1995 Dec, 177 (23) : 6894-901.

Weiterhin ist aus der EP277674 ein mikrobiologisches Verfahren zur terminalen Hydroxylierung von apolaren aliphatischen Verbindungen mit 6 bis 12 C-Atomen, wie die Herstellung von 1-Octanol, mittels Mikroorganismen der Gattung *Pseudomonas putida,* die gegen apolare Phasen resistent sind, bekannt, wobei unter anderem ein das alkL-Gen aufweisende Plasmid pGEc47 eingesetzt wird, welches ebenfalls beide alk-Operons aus *Pseudomonas putida* trägt. Die Kontrolle des alkL-Gens liegt unter der Kontrolle des nativen Operon-Promoters und wird daher zusammen mit alkB, alkF, alkG, alkH, alkJ und alkK transkribiert und translatiert.

Die WO2002022845 beschreibt ein Verfahren zur Herstellung von N-Benzyl-4-Hydroxypiperidin durch Hydroxylierung von N-Benzyl-4-Piperidin durch *E. coli* Zellen, die das oben genannte Plasmid pGEc47 tragen.

Die EP0502524 beschreibt ein mikrobiologisches Verfahren zur terminalen Hydroxylierung von Ethylgruppen an aromatischen 5-oder 6-Ring-Heterocyclen mit Hilfe der Produktion verschiedener Genprodukte der alk-Operons, so z. B. über das Plasmid pGEc41, welches für die Genprodukte von alkB, alkG, alkH, alkT und alkS, aber nicht von alkL kodiert. Die gleiche Anmeldung, beschreibt darüber hinaus ein Plasmid pGMK921, das wie pGEc41 die Gene für alkB, alkG, alkH, alkT und alkS - aber nicht alkL - enthält, deren Expression aber nicht nur durch Alkaninduktion vom nativen Promotor, sondern auch durch IPTG-Induktion vom tac-Promotor aus möglich ist (vgl. auch US5306625).

Schneider et al. beschreiben in Appl Environ Microbiol. 1998 Oct; 64(10):3784-90 eine Biokonversion von gesättigten Fettsäuren zu deren ω-1-, ω-2- und ω-3-Hydroxy-Fettsäuren in *E. coli* mit Hilfe einer Cytochrom P-450BM-3 Monooxygenase und dem oben genannte Plasmid pGEc47.

Favre-Bulle et al. beschreiben in Nature Bio/Technology 9, 367-371 (April 1991) ein Verfahren zur Herstellung von 1-Oktansäure durch Biotransformation von Oktan mit einem als Biokatalysator eingesetzten pGEc47 tragenden *E. coli*-Bakterium. Beide alk-Operons werden im beschriebenen Verfahren komplett exprimiert.

Den gleichen Ansatz verfolgen Rothen et al., in Biotechnol Bioeng. 1998 May 20;58(4):356-65.

Nachteil des beschriebenen Standes der Technik ist, dass Genprodukte, die keinen wesentlichen Beitrag zu dem gewünschten Oxidationsprozess leisten, überflüssigerweise von der als Biokatalysator eingesetzten Zelle produziert werden und somit deren Leistungsfähigkeit herabsetzen.

Darüber hinaus bergen die unnötig co-synthetisierten alk-Genprodukte gegebenenfalls unerwünschte Enzymaktivitäten, die der gewünschten Produktbildung abträglich sind, etwa dadurch, dass Zwischenprodukt in unerwünschte Nebenprodukte abfließt. Bei gewünschter ω-Hydroxylierung eines organischen Restes führt das alkJ-Genprodukt zur Bildung des korrespondierenden Aldehyds. Bei beispielsweise gleichzeitiger Anwesenheit des alkH-Genprodukts wird der entstehende Aldehyd weiter zur Carbonsäure oxidiert.

So werden in der EP0502524 für die Erzeugung des gewünschten hydroxylierten Verfahrensproduktes lediglich die Genprodukte von alkB, alkG und alkT benötigt, wodurch z.B. die Gene alkF, alkJ, alkH und alkS überflüssig sind. Nachteilig ist hier außerdem, dass die Synthese weiterer alk-Genprodukte hohe Anforderungen an die Stoffwechselkapazität des Wirts stellt. AlkJ z.B. ist ein FAD-haltiges Enzym (Chen et al., J. Bacteriol, (1995), 6894-6901). Der FAD-Pool des Wirtes ist jedoch schon durch die unverzichtbare Produktion von alkT belastet, welches ebenfalls FAD enthält. Da die FAD-Synthese-Kapazität in z.B. *E. coli* begrenzt ist und ebenfalls für den existentiellen Zellstoffwechsel benötigt wird, wird die Zelle bei unnötiger alkJ-Produktion vermeidbar belastet.

Des Weiteren sind die Genprodukte von alkB, alkJ und alkH Cytoplasma-Membran-ständig oder Cytoplasma-Membran-assoziiert. In diesem Bereich ist auch die Atmungskette angesiedelt. Eine übermäßige Produktion von Membranproteinen führt von Veränderungen in der Zellmembran bis zur Ablösung von Membranvesikeln, die ins Cytosol abwandern. (Nieboer et al., Molecular Microbiology (1993) 8(6), 1039-1051)
Dies führt letztlich zu einer vorzeitigen Lyse der Zellen (Wubbolts et al., Biotechnolgy and Bioengineering (1996), Vol 52, 301-308), erst recht in der für industrielle Prozesse unverzichtbaren Hochzelldichte-Fermentation.

Analog werden in Schneider et al. die Genprodukte von alkB, alkF, alkG, alkH, alkJ, alkK, alkS und alkT überflüssigerweise mit synthetisiert, da das eigentliche zur gewünschten Reaktion eingesetzte Enzym die Cytochrom P-450BM-3 Monooxygenase ist.

Im Hinblick auf industrielle Prozesse ist die Verwendung von plasmidkodierten Stoffwechselwegen schwierig. Mit steigender Größe der Fermentervolumen wird die Verwendung von Antibiotika zur Aufrechterhaltung eines Selektionsdrucks, der die Plasmidstabilität verbessert, zum einen sehr teuer und zum anderen abwasserkritisch. Großfermentationen laufen daher fast immer ohne jede Antibiotikumsgabe ab.

Um die genetische Stabilität des artifiziellen oxidativen Stoffwechselweges dennoch zu gewährleisten ist eine Integration der verwendeten Gene ins Genom des Wirtsorganismus wünschenswert. Ein solcher Ansatz gelingt umso besser, je kleiner das zu integrierende Genkonstrukt ist. Da das hier betrachtete Minimal-Gen-Set alkBGTL schon eine beachtliche Größe hat, ist jede weitere, nicht unbedingt notwendige Nukleotidsequenz zu vermeiden.

Neben der Reduktion des Umfangs der nötigen molekularbiologischen Arbeiten und der Erhöhung ihrer Erfolgswahrscheinlichkeit ist ein möglichst kleines Konstrukt auch der genomischen Stabilität des Wirtsorganismus zuträglich.

Aufgabe der Erfindung war es, ein Verfahren bereitzustellen, welches zumindest einen der genannten Nachteile des Standes der Technik zu überwinden vermag.

<u>Beschreibung der Erfindung</u>

Überraschenderweise wurde gefunden, dass das im Folgenden beschriebene Verfahren und die gentechnisch veränderten Zellen einen Beitrag zur Lösung der gestellten Aufgabe leisten.

Gegenstand der vorliegenden Erfindung sind daher ein Verfahren zur Herstellung einer oxidierten, organischen Substanz unter Nutzung eines alkL-Genproduktes wie in Anspruch 1 beschrieben sowie die in diesem Verfahren eingesetzten, rekombinanten Zellen.

Ein weiterer Gegenstand der Erfindung ist die Verwendung eines alkL-Genproduktes zur Erhöhung der Oxidationsrate.

Vorteile sind die optimale Ausnutzung der im Verfahren vorhandenen Ressourcen, beispielsweise hinsichtlich des Zellstoffwechsels, insbesondere unter Hochzelldichtefermentations-Bedingungen.

Die vorliegende Erfindung beschreibt ein Verfahren zur Oxidation einer organischen Substanz unter Einsatz mindestens eines oxidierenden Enzyms und mindestens eines alkL-Genproduktes, dadurch gekennzeichnet, dass das alkL-Genprodukt unabhängig von mindestens einem weiteren, durch das das alkL-Gen enthaltende alk-Operon kodierten Genprodukt bereitgestellt wird, dadurch gekennzeichnet, dass das weitere Genprodukt ausgewählt ist aus mindestens einem aus der Gruppe bestehend aus AlkF, AlkG, und AlkH und AlkJ, und dass das alkL-Genprodukt ausgewählt ist aus der Gruppe bestehend aus Proteine kodiert durch die alkL-Gene aus Pseudomonas putida GPO1 und P1, welche wiedergegebenen werden durch Seq ID Nr. 1 und Seq ID Nr. 3 und Proteine mit Polypeptidsequenz Seq ID Nr. 2 oder Seq ID Nr. 4.

Die im Zusammenhang mit dieser Erfindung beschriebenen alk-Gene kodieren für Proteinsequenzen, die analog als AlkX bezeichnet werden. Werden mehrere Gene alkX, alkY und alkZ gleichzeitig beschrieben so wird die Nomenklatur alkXYZ bzw. analog bei Proteinen AlkXYZ verwendet.

Unter dem Begriff "Oxidation einer organischen Substanz" wird im Zusammenhang mit der vorliegenden Erfindung beispielsweise eine Hydroxylierung oder Epoxidierung, die Umsetzung eines Alkohols zu einem Aldehyd oder Keton, die Umsetzung eines Aldehydes zu einer Carbonsäure oder die Hydratisierung einer Doppelbindung verstanden. Ebenso sind hierunter auch mehrstufige Oxidationsprozesse zusammengefasst, wie man sie insbesondere durch den Einsatz mehrerer oxidierender Enzyme erreichen kann, wie beispielsweise die Hydroxylierung eines Alkyl-Restes an mehren Stellen, z.B. $\omega$-ständig und $\omega$-1-ständig, katalysiert durch verschiedene Monooxygenasen. Unter dem Begriff "unter Einsatz mindestens eines oxidierenden Enzyms und mindestens eines alkL-Genproduktes" wird im Zusammenhang mit der vorliegenden Erfindung das gezielte Bereitstellen der Enzyme und Genprodukte verstanden, und zwar in einer Form, wie man jedes einzelne Enzym oder Genprodukt für sich selber betrachtet in der freien Natur nicht vorfindet. Dies kann beispielsweise durch heterologe Produktion oder Überproduktion der eingesetzten Proteine in einer Zelle oder durch Bereitstellen von mindestens teilweise aufgereinigten Proteinen erfolgen,; eingeschlossen sind hier aber auch eine geänderte Umgebung im Vergleich zu dem in freier Natur vorkommenden Enzym, etwa in der Gestalt, dass die natürliche Zelle beinhaltend das Enzym modifiziert wurde, so dass sie beispielsweise bestimmte andere Proteine in geänderter Form, wie beispielsweise abgeschwächt oder verstärkt oder mit Punktmutationen versehen, produziert.

Unter dem Begriff "alkL-Genprodukt" werden im Zusammenhang mit der vorliegenden Erfindung Proteine verstanden, die mindestens eine der nachfolgenden beiden Bedingungen erfüllen:

1.) Das Protein wird als ein Mitglied der Superfamilie der OmpW-Proteine (Proteinfamilie 3922 in der "Conserved Domain Database" (CDD) des "National Center for Biotechnology Information" (NCBI)) identifiziert, wobei diese Zuordnung durch ein Alignment der Aminosäuresequenz des Proteins mit den in der CDD des NCBI vorhandenen Datenbankeinträgen, die bis zum 22.03.2010 hinterlegt wurden, unter Nutzung der Standardsuchparameter, einem E-Wert (englisch "e-value") kleiner 0,01 und unter Verwendung des Algorithmus "blastp 2.2.23+", erfolgt,
2.) bei einer Suche nach in der betreffenden Aminosäuresequenz enthaltenen konservierten Proteindomänen in der NCBI CDD (Version 2.20) mittels RPS-BLAST wird die Präsenz der konservierten Domäne "OmpW, Outer membrane protein W" (COG3047) mit einem E-Wert (englisch "e-value") kleiner 1 x $10^{-5}$ festgestellt (englisch "domain hit").

Unter dem Begriff "unabhängig von mindestens einem weiteren, durch das das alkL-Gen enthaltende alk-Operon kodierten Genprodukt bereitgestellt" wird im Zusammenhang mit der vorliegenden Erfindung eine Bereitstellung des alkL-Genproduktes verstanden, welche von mindestens einem weiteren alk-Genprodukt, welches in natürlich vorkommender Form an das Entstehen des alkL-Genproduktes gekoppelt ist, unabhängig ist. Beispielsweise sind in einem Operon umfassend die Gene alkBFGHJKL die alk-Genprodukte von jeweils alkBFGHJ und K an die Entstehung des alkL-Genproduktes gekoppelt, da diese über denselben Promotor bereitgestellt werden.

Alle angegebenen Prozent (%) sind wenn nicht anders angegeben Massenprozent.

Das erfindungsgemäße Verfahren lässt sich in Abhängigkeit des eingesetzten oxidierenden Enzyms für die Oxidation jeglicher organischer Substanzen einsetzen, welche von diesem oxidierenden Enzym als Substrat akzeptiert werden; bevorzugte organische Substanzen sind ausgewählt aus der Gruppe enthaltend, bevorzugt bestehend aus, verzweigte oder unverzweigte, bevorzugt unverzweigte, gesättigte oder ungesättigte, bevorzugt gesättigte, gegebenenfalls substituierte
Alkane, Alkene, Alkine, Alkohole, Aldehyde, Ketone, Carbonsäuren, Carbonsäureester, Amine und Epoxide,
wobei diese bevorzugt 3 bis 22, insbesondere 6 bis 18, weiterhin bevorzugt 8 bis 14, insbesondere 12 Kohlenstoffatome aufweisen.

Besonders bevorzugte organische Substanzen im erfindungsgemäßen Verfahren sind ausgewählt aus der Gruppe enthaltend, bevorzugt bestehend aus,
Carbonsäuren und deren korrespondierenden Ester, insbesondere mit 3 bis 22, bevorzugt 6 bis 18, besonders bevorzugt 8 bis 14, Kohlenstoffatomen insbesondere Carbonsäuren der Alkane, insbesondere unverzweigte Carbonsäuren der Alkane, insbesondere Laurinsäure und ihre Ester, insbesondere Laurinsäuremethyl- und Laurinsäureethylester, Decansäure, Decansäureester, Myristinsäure und Myristinsäureester,

unsubstituierte Alkane mit 3 bis 22, bevorzugt 6 bis 18, besonders bevorzugt 8 bis 14 Kohlenstoffatomen, bevorzugt unverzweigte, insbesondere ausgewählt aus der Gruppe enthaltend, bevorzugt bestehend aus, Octan, Decan, Dodecan und Tetradecan,

unsubstituierte Alkene mit 3 bis 22, bevorzugt 6 bis 18, besonders bevorzugt 8 bis 14, Kohlenstoffatomen, bevorzugt unverzweigte, insbesondere ausgewählt aus der Gruppe enthaltend, bevorzugt bestehend aus, trans-Oct-1-en, trans-Non-1-en, trans-Dec-1-en, trans-Undec-1-en, trans-Dodec-1-en, trans-Tridec-1-en, trans-Tetrade-1-cen, cis-Oct-1-en, cis-Non-1-en, cis-Dec-1-en, cis-Undec-1-en, cis-Dodec-1-en, cis-Tridec-1-en, cis-Tetrade-1-cen, trans-Oct-2-en, trans-Non-2-en, trans-Dec-2-en, trans-Undec-2-en, trans-Dodec-2-en, trans-Tridec-2-en und trans-Tetradec-2-en, trans-Oct-3-en, trans-Non-3-en, trans-Dec-3-en, trans-Undec-3-en, trans-Dodec-3-en, trans-Tridec-3-en und trans-Tetradec-3-en, trans-Oct-4-en, trans-Non-4-en, trans-Dec-4-en, trans-Undec-4-en, trans-Dodec-4-en, trans-Tridec-4-en, trans-Tetradec-4-en, trans-Dec-5-en, trans-Undec-5-en, trans-Dodec-5-en, trans-Tridec-5-en, trans-Tetradec-5-en, trans-Dodec-6-en, trans-Tridec-6-en, trans-Tetradec-6-en und trans-Tetradec-7-en, besonders bevorzugt bestehend aus, trans-Oct-1-en, trans-Dec-1-en, trans-Dodec-1-en, trans-Tetrade-1-cen, cis-Oct-1-en, cis-Dec-1-en, cis-Dodec-1-en, cis-Tetrade-1-cen, trans-Oct-2-en, trans-Dec-2-en, trans-Dodec-2-en und trans-Tetradec-2-en, trans-Oct-3-en, trans-Dec-3-en, trans-Dodec-3-en und trans-Tetradec-3-en, trans-Oct-4-en, trans-Dec-4-en, trans-Dodec-4-en, trans-Tetradec-4-en, trans-Dec-5-en, trans-Dodec-5-en, trans-Tetradec-5-en, trans-Dodec-6-en, trans-Tetradec-6-en und trans-Tetradec-7-en,

unsubstituierte, einwertige Alkohole mit 3 bis 22, bevorzugt 6 bis 18, besonders bevorzugt 8 bis 14, Kohlenstoffatomen, bevorzugt unverzweigte, insbesondere ausgewählt aus der Gruppe enthaltend, bevorzugt bestehend aus, 1-Octanol, 1-Nonanol, 1-Decanol,1-Undecanol, 1-Dodecanol, 1-Tridecanol und 1-Tetradecanol,
besonders bevorzugt bestehend aus 1-Octanol, 1-Decanol, 1-Dodecanol und 1-Tetradecanol

unsubstituierte Aldehyde mit 3 bis 22, bevorzugt 6 bis 18, besonders bevorzugt 8 bis 14, Kohlenstoffatomen, bevorzugt unverzweigte, insbesondere ausgewählt aus der Gruppe enthaltend, bevorzugt bestehend aus, Octanal, Nonanal, Decanal, Dodecanal und Tetradecanal,

unsubstituierte, einwertige Amine mit 3 bis 22, bevorzugt 6 bis 18, besonders bevorzugt 8 bis 14, Kohlenstoffatomen, bevorzugt unverzweigte, insbesondere ausgewählt aus der Gruppe enthaltend, bevorzugt bestehend aus, 1-Amino-Octan, 1-Amino-Nonan, 1-Amino-Decan, 1-Amino-Undecan, 1-Amino-Dodecan, 1-Amino-Tridecan und 1-Amino-Tetradecan,
besonders bevorzugt bestehend aus, 1-Amino-Octan, 1-Amino-Decan, 1-Amino-Dodecan und 1-Amino-Tetradecan,
sowie substituierte Verbindungen, die insbesondere als weitere Substituenten einen oder mehrer Hydroxy-, Amin-, Keto-, Carboxyl-, Cyclopropylreste oder Epoxy-Funktionen tragen, insbesondere ausgewählt aus der Gruppe enthaltend, bevorzugt bestehend aus, 1,8-Octandiol, 1,9-Nonandiol, 1,10-Decandiol, 1,11-Undecandiol, 1,12-Dodecandiol, 1,13-tridecandiol, 1,14-Tetradecandiol, 8-Amino-[1-Octanol], 9-Amino-[1-Nonanol], 10-Amino-[1-Dodecanol], 11-Amino-[1- Undecanol], 12-Amino-[1-Dodecanol], 13-Amino-[1-Tridecanol], 14-Amino-[1-Tetradecanol], 8-Hydroxy-[1-Octanal], 9-hydroxy-[1-Nonanal], 10-Hydroxy-[1-Decanal], 11-Hydroxy-[1-Undecanal], 12-Hydroxy-[1-Dodecanal], 13-Hydroxy-[1-Tridecanal], 14-Hydroxy-[1-Tetradecanal], 8-Amino-[1-Octanal], 9-Amino-[1-Nonanal], 10-Amino-[1-Decanal], 11-Amino-[1-Undecanal], 12-Amino-[1-Dodecanal], 13-Amino-[1-Tridecanal], 14-Amino-[1-Tetradecanal],
8-Hydroxy-[1-Octansäure], 9-Hydroxy-[1-Nonansäure], 10-Hydroxy-[1-Decansäure], 11-Hydroxy-[1-Undecansäure], 12-Hydroxy-[1-Dodecansäure], 13-Hydroxy-[1-Undecansäure], 14-Hydroxy-[1-Tetradecansäure], 8-Hydroxy-[1-Octansäure-Methylester], 9-Hydroxy-[1-Nonansäure-Methylester], 10-Hydroxy-[1-Decansäure-Methylester], 11-Hydroxy-[1-Undecansäure-Methylester], 12-Hydroxy-[1-Dodecansäure-Methylester], 13-Hydroxy-[1-Undecansäure-Methylester], 14-Hydroxy-[1-Tetradecansäure-Methylester], 8-Hydroxy-[1-Octansäure-Ethylester], 9-Hydroxy-[1-Nonansäure-Ethylester], 10-Hydroxy-[1-Decansäure-Ethylester], 11-Hydroxy-[1-Undecansäure-Ethylester], 12-Hydroxy-[1-Dodecansäure-Ethylester], 13-Hydroxy-[1-Undecansäure-Ethylester] und 14-Hydroxy-[1-Tetradecansäure-Ethylester],
besonders bevorzugt bestehend aus, 1,8-Octandiol, 1,10-Decandiol, 1,12-Dodecandiol, 1,14-Tetradecandiol, 8-Amino-[1-Octanol], 10-Amino-[1-Dodecanol], 12-Amino-[1-Dodecanol], 14-Amino-[1-Tetradecanol], 8-Hydroxy-[1-Octanal], 10-Hydroxy-[1-Decanal], 12-Hydroxy-[1-Dodecanal], 14-Hydroxy-[1-Tetradecanal], 8-Amino-[1-Octanal], 10-Amino-[1-Decanal], 12-Amino-[1-Dodecanal], 14-Amino-[1-Tetradecanal],
8-Hydroxy-[1-Octansäure], 10-Hydroxy-[1-Decansäure], 12-Hydroxy-[1-Dodecansäure], 14-Hydroxy-[1-Tetradecansäure], 8-Hydroxy-[1-Octansäure-Methylester], 10-Hydroxy-[1-Decansäure-Methylester], 12-Hydroxy-[1-Dodecansäure-Methylester], 14-Hydroxy-[1-Tetradecansäure-Methylester], 8-Hydroxy-[1-Octansäure-Ethylester], 10-Hydroxy-[1-Decansäure-Ethylester], 12-Hydroxy-[1-Dodecansäure-Ethylester] und 14-Hydroxy-[1-Tetradecansäure-Ethylester],
wobei Laurinsäure und ihre Ester, insbesondere Laurinsäuremethyl- und Laurinsäureethylester, besonders bevorzugt sind.

Mit dem erfindungsgemäßen Verfahren lassen sich in Abhängigkeit des eingesetzten oxidierenden Enzyms und der eingesetzten organischen Substanz verschiedene Oxidationsprodukte herstellen, insbesondere Alkohole, Aldehyde, Ketone und Carbonsäuren.

Diese Oxidationsprodukte lassen sich beispielsweise durch das erfindungsgemäße Verfahren erhalten durch die Umsetzung einer im Folgenden gelisteten organischen Substanz zu:

- Alkan/Alken/Alkin zu Alkohol (beispielsweise mit einer Monooxygenase)
- Alkohol zu Aldehyd (beispielsweise mit einer Alkoholdehydrogenase oder Alkoholoxidase)
- Alkohol zu Keton (beispielsweise mit einer Alkoholdehydrogenase oder Alkoholoxidase)
- Aldehyd zu Carbonsäure (beispielsweise mit einer Aldehyddehydrogenase)
- Epoxid zum Cyanhydrin (beispielsweise mit einer Halohydrin-Dehalogenase)

In diesem Zusammenhang ist die Herstellung von Alkoholen und Aldehyden bevorzugt von Alkoholen, insbesondere von ω-Alkoholen, ganz besonders von ω-Hydroxy-Carbonsäuren mit dem erfindungsgemäßen Verfahren, insbesondere in Form einer Hydroxylierungsreaktion, bevorzugt.

In dem erfindungsgemäßen Verfahren lassen sich organische Substanzen, insbesondere Carbonsäuren und Carbonsäureester, vorteilhaft an der ω-Position oxidieren.

In dem erfindungsgemäßen Verfahren lassen sich alle dem Fachmann bekannten oxidierenden Enzyme einsetzen, da die Funktion des bereitgestellten alkL-Genproduktes hiervon unabhängig ist. Solche Enzyme sind dem Fachmann hinlänglich unter dem Begriff Oxidoreduktase bekannt und sind in der Enzymklasse EC 1.X.X.X der systematischen Nomenklatur der Enzymkommission der International Union of Biochemistry and Molecular Biology zu finden.

Bevorzugt werden in dem erfindungsgemäßen Verfahren als oxidierendes Enzym eine Alkanmonooxygenase, eine Xylolmonooxygenase, eine Aldehyddehydrogenase, eine Alkoholoxidase oder eine Alkoholdehydrogenase, vorzugsweise eine Alkanmonooxygenase eingesetzt.

Ein geeignetes Gen für eine Xylolmonooxygenase ist beispielsweise das xylM- oder das xylA-Gen, wobei ein Plasmid beinhaltend diese beiden Gene die GENBANK-Accession-Nr. M37480 besitzt.

Eine besonders bevorzugte Alkanmonooxygenase ist in diesem Zusammenhang dadurch gekennzeichnet, dass sie eine Cytochrom-P450-Monoxygenasen, insbesondere eine Cytochrom-P450-Monoxygenasen aus Hefen, insbesondere *Pichia, Yarrowia* und *Candida,* beispielsweise aus *Candida tropicalis oder Candida maltosa,* oder aus Pflanzen, beispielsweise aus der *Cicer arietinum L.,* oder aus Säugern, beispielsweise aus *Rattus norvegicus,* insbesondere CYP4A1, ist. Die Gensequenzen geeigneter Cytochrom-P450-Monoxygenasen aus *Candida tropicalis* sind beispielsweise in WO-A-00/20566 offenbart, während die Gensequenzen geeigneter Cytochrom-P450-Monoxygenasen aus der Kichererbse beispielsweise Barz et al. in "Cloning and characterization of eight cytochrome P450 cDNAs from chickpea (Cicer arietinum L.) cell suspension cultures", Plant Science, Vol. 155, Seiten 101-108 (2000) entnommen werden können.

Eine weitere bevorzugte Alkanmonooxygenase wird vom alkB-Gen des alk-Operons aus *Pseudomonas putida* GPo1 kodiert.

Die Isolierung der alkB-Gensequenz ist beispielsweise von van Beilen et al. in "Functional Analysis of Alkane Hydroxylases from Gram-Negative and Gram-Positive Bacteria", Journal of Bacteriology, Vol. 184 (6), Seiten 1.733-1.742 (2002) beschrieben. Weitere Homologe des alkB-Gens können auch van Beilen et al. in "Oil & Gas Science and Technology", Vol. 58 (4), Seiten 427-440 (2003) entnommen werden.

Weiterhin bevorzugte Alkanmonooxygenasen sind solche *alkB*-Genprodukte, welche kodiert werden von *alkB*-Genen aus Organismen ausgewählt aus der Gruppe der gram-negativen Bakterien, insbesondere aus der Gruppe der Pseudomonaden, dort aus der Gattung *Pseudomonas,* besonders *Pseudomonas mendocina,* der Gattung *Oceanicaulis,* bevorzugt *Oceanicaulis alexandrii* HTCC2633, der Gattung *Caulobacter,* bevorzugt *Caulobacter* sp. K31, der Gattung *Marinobacter,* bevorzugt *Marinobacter aquaeolei,* besonders bevorzugt *Marinobacter aquaeolei* VT8, der Gattung *Alcanivorax,* bevorzugt *Alcanivorax borkumensis,* der Gattung *Acetobacter, Achromobacter, Acidiphilium, Acidovorax, Aeromicrobium, Alkalilimnicola, Alteromonadales, Anabaena, Aromatoleum, Azoarcus, Azospirillum, Azotobacter, Bordetella, Bradyrhizobium, Burkholderia, Chlorobium, Citreicella, Clostridium, Colwellia, Comamonas, Conexibacter, Congregibacter, Corynebacterium, Cupriavidus, Cyanothece, Delftia, Desulfomicrobium, Desulfonatronospira, Dethiobacter, Dinoroseobacter, Erythrobacter, Francisella, Glaciecola, Gordonia, Grimontia, Hahella, Haloterrigena, Halothiobacillus, Hoeflea, Hyphomonas, Janibacter, Jannaschia, Jonquetella, Klebsiella, Legionella, Limnobacter, Lutiella, Magnetospirillum, Mesorhizobium, Methylibium, Methylobacterium, Methylophaga, Mycobacterium, Neisseria, Nitrosomonas, Nocardia, Nostoc, Novosphingobium, Octadecabacter, Paracoccus, Parvibaculum, Parvularcula, Peptostreptococcus, Phaeobacter, Phenylobacterium, Photobacterium, Polaromonas, Prevotella, Pseudoalteromonas, Pseudovibrio, Psychrobacter, Psychroflexus, Ralstonia, Rhodobacter, Rhodococcus, Rhodoferax, Rhodomicrobium, Rhodopseudomonas, Rhodospirillum, Roseobacter, Roseovarius, Ruegeria, Sagittula, Shewanella, Silicibacter, Stenotrophomonas, Stigmatella, Streptomyces, Sulfitobacter, Sulfurimonas, Sulfurovum, Synechococcus, Thalassiobium, Thermococcus, Thermomonospora, Thioalkalivibrio, Thiobacillus, Thiomicrospira, Thiomonas, Tsukamurella, Vibrio* oder *Xanthomonas,* wobei die aus *Alcanivorax borkumensis, Oceanicaulis alexandrii HTCC2633, Caulobacter sp. K31 und Marinobacter aquaeolei VT8* besonders bevorzugt sind. Es ist in diesem Zusammenahng vorteilhaft wenn zusätzlich zu AlkB alkG- und alkT-Genprodukte bereitgestellt werden; diese können entweder die Genprodukte isolierbar aus dem das alkB-Genprodukt beisteuernden Organismus sein, oder aber das alkG und alkT aus *Pseudomonas putida GPo1* sein.

Eine bevorzugte Alkoholdehydrogenase ist beispielsweise das vom *alk*J-Gen kodierte Enzym (EC 1.1.99.8), insbesondere das vom *alk*J-Gen kodierte Enzym aus *Pseudomonas putida* GPo1 (van Beilen et al., Molecular Microbiology, (1992) 6(21), 3121-3136). Die Gensequenzen der *alk*J-Gene aus *Pseudomonas putida* GPo1, *Alcanivorax borkumensis, Bordetella parapertussis, Bordetella bronchiseptica* oder aus *Roseobacter denitrificans* können beispielsweise der KEGG-Gendatenbank (Kyoto Encyclopedia of Genes and Genomes) entnommen werden. Weiterhin bevorzugte Alkoholdehydrogenasen sind solche, die kodiert werden von *alkJ*-Genen aus Organismen ausgewählt aus der Gruppe der gram-negativen Bakterien, insbesondere aus der Gruppe der Pseudomonaden, dort aus der Gattung *Pseudomonas,* besonders *Pseudomonas mendocina,* der Gattung *Oceanicaulis,* bevorzugt *Oceanicaulis alexandrii* HTCC2633, der Gattung *Caulobacter,* bevorzugt *Caulobacter* sp. K31, der Gattung *Marinobacter,* bevorzugt *Marinobacter aquaeolei,* besonders bevorzugt *Marinobacter aquaeolei* VT8, der Gattung *Alcanivorax,* bevorzugt *Alcanivorax borkumensis,* der Gattung *Acetobacter, Achromobacter, Acidiphilium, Acidovorax, Aeromicrobium, Alkalilimnicola, Alteromonadales, Anabaena, Aromatoleum, Azoarcus, Azospirillum, Azotobacter, Bordetella, Bradyrhizobium, Burkholderia, Chlorobium, Citreicella, Clostridium, Colwellia, Comamonas, Conexibacter, Congregibacter, Corynebacterium, Cupriavidus, Cyanothece, Delftia, Desulfomicrobium, Desulfonatronospira, Dethiobacter, Dinoroseobacter, Erythrobacter, Francisella, Glaciecola, Gordonia, Grimontia, Hahella, Haloterrigena, Halothiobacillus, Hoeflea, Hyphomonas, Janibacter, Jannaschia, Jonquetella, Klebsiella, Legionella, Limnobacter, Lutiella, Magnetospirillum, Mesorhizobium, Methylibium, Methylobacterium, Methylophaga, Mycobacterium, Neisseria, Nitrosomonas, Nocardia, Nostoc, Novosphingobium, Octadecabacter, Paracoccus, Parvibaculum, Parvularcula, Peptostreptococcus, Phaeobacter, Phenylobacterium, Photobacterium, Polaromonas, Prevotella, Pseudoalteromonas, Pseudovibrio, Psychrobacter, Psychroflexus, Ralstonia, Rhodobacter, Rhodococcus, Rhodoferax, Rhodomicrobium, Rhodopseudomonas, Rhodospirillum, Roseobacter, Roseovarius, Ruegeria, Sagittula, Shewanella, Silicibacter, Stenotrophomonas, Stigmatella, Streptomyces, Sulfitobacter, Sulfurimonas, Sulfurovum, Synechococcus, Thalassiobium, Thermococcus, Thermomonospora, Thioalkalivibrio, Thiobacillus, Thiomicrospira, Thiomonas, Tsukamurella, Vibrio* oder *Xanthomonas.*

Bevorzugte in dem erfindungsgemäßen Verfahren eingesetzte alkL-Genprodukte sind dadurch gekennzeichnet, dass die Produktion des alkL-Genproduktes im nativen Wirt durch Dicyclopropylketon induziert wird; in diesem Zusammenhang ist weiterhin bevorzugt, dass die Expression des alkL-Gens als Teil einer Gruppe von Genen, beispielsweise in einem Regulon wie etwa einem Operon, stattfindet.

In dem erfindungsgemäßen Verfahren eingesetzte alkL-Genprodukte werden bevorzugt kodiert von alkL-Genen aus Organismen ausgewählt aus der Gruppe der gram-negativen Bakterien, insbesondere der Gruppe enthaltend, bevorzugt bestehend aus, Pseudomonaden, besonders *Pseudomonas putida,* insbesondere *Pseudomonas putida* GPo1 und P1, *Azotobacter, Desulfitobacterium, Burkholderia,* bevorzugt *Burkholderia cepacia, Xanthomonas, Rhodobacter, Ralstonia, Delftia* und *Rickettsia,* die Gattung *Oceanicaulis,* bevorzugt *Oceanicaulis alexandriii* HTCC2633, die Gattung *Caulobacter,* bevorzugt *Caulobacter* sp. K31, die Gattung *Marinobacter,* bevorzugt *Marinobacter aquaeolei,* besonders bevorzugt *Marinobacter aquaeolei* VT8 und die Gattung *Rhodopseudomonas.*

Es ist vorteilhaft, wenn das alkL-Genprodukt aus einem anderen Organismus stammt, als das erfindungsgemäß eingesetzte oxidierende Enzym.

In diesem Zusammenhang sind ganz besonders bevorzugte alkL-Genprodukte kodiert durch die alkL-Gene aus *Pseudomonas putida* GPo1 und P1, welche wiedergegeben werden durch Seq ID Nr. 1 und Seq ID Nr. 3, sowie Proteine mit Polypeptidsequenz Seq ID Nr. 2 oder Seq ID Nr. 4

Wobei unter 100% Aktivität des Bezugsprotein die Erhöhung der Aktivität der als Biokatalysator verwendeten Zellen, also die pro Zeiteinheit umgesetzte Stoffmenge bezogen auf die eingesetzte Zellmenge (Units pro Gramm Zelltrockenmasse (cell dry weight) [U/gCDW]) im Vergleich zur Aktivität des Biokatalysators ohne Anwesenheit des Bezugsproteins verstanden wird, und zwar in einem System, wie es in den Ausführungsbeispielen beschrieben wird, bei dem als oxidierende Enzyme die Genprodukte von alkBGT aus *P. putida* GPo1 zur Umsetzung von Laurinsäuremethylester zu 12-Hydroxylaurinsäuremetylester in einer *E. coli*-Zelle eingesetzt werden. Eine Methode der Wahl zur Bestimmung der Oxidationsrate ist den Ausführungsbeispielen zu entnehmen.

Für die Definition des Units gilt hier die in der Enzymkinetik übliche Definition. 1 Unit Biokatalysator setzt 1 μmol Substrat in einer Minute zum Produkt um.
1 U = 1 μmol/min

Änderungen von Aminosäureresten einer gegebenen Polypeptidsequenz, die zu keinen wesentlichen Änderungen der Eigenschaften und Funktion des gegebenen Polypeptides führen, sind dem Fachmann bekannt. So können beispielsweise manche Aminosäuren oft problemlos gegeneinander ausgetauscht werden; Beispiele für solche geeigneten Aminosäuresubstitutionen sind: Ala gegen Ser; Arg gegen Lys; Asn gegen Gln oder His; Asp gegen Glu; Cys gegen Ser; Gln gegen Asn; Glu gegen Asp; Gly gegen Pro; His gegen Asn oder Gln; Ile gegen Leu oder Val; Leu gegen Met oder Val; Lys gegen Arg oder Gln oder Glu; Met gegen Leu oder Ile; Phe gegen Met oder Leu oder Tyr; Ser gegen Thr; Thr gegen Ser; Trp gegen Tyr; Tyr gegen Trp oder Phe; Val gegen Ile oder

Leu. Ebenso ist bekannt, dass Änderungen besonders am N- oder C-Terminus eines Polypeptides in Form von beispielsweise Aminosäureinsertionen oder -deletionen oft keinen wesentlichen Einfluss auf die Funktion des Polypeptides ausüben.

Ein erfindungsgemäß bevorzugtes Verfahren ist dadurch gekennzeichnet, dass das weitere Genprodukt ausgewählt ist aus mindestens einem aus der Gruppe bestehend aus
AlkF, AlkG, und AlkHinsbesondere bestehend aus AlkF, AlkG,und AlkH,
wobei weitere Genprodukte insbesondere ausgewählt sind aus der Gruppe enthaltend, bevorzugt bestehend aus den Genkombinationen: alkFH, alkGH, und alkFGH,.

Es ist für das erfindungsgemäße Verfahren vorteilhaft, wenn das oxidierende Enzym und das alkL-Genprodukt durch einen Mikroorganismus bereitgestellt werden. Dabei können die beiden Enzyme jeweils getrennt in jeweils einem Mikroorganismus oder gemeinsam in einem Mikroorganismus bereitgestellt werden, wobei letzteres bevorzugt ist. Daher ist ein bevorzugtes erfindungsgemäßes Verfahren dadurch gekennzeichnet, dass es in mindestens einem Mikroorganismus oder in einem den mindestens einen Mikroorganismus, der das oxidierende Enzym und das alkL-Genprodukt bereitstellt, umgebenden Medium durchgeführt wird.

In diesem Zusammenhang ist es bevorzugt, dass das oxidierende Enzym und das alkL-Genprodukt in dem mindestens einen Mikroorganismus rekombinant bereitgestellt werden.

Die nun folgenden Ausführungen zur rekombinanten Produktion beziehen sich sowohl auf das oxidierende Enzym als auch auf das alkL-Genprodukt.

Grundsätzlich lässt sich eine rekombinante Produktion dadurch erzielen, dass man die Kopienzahl der Gensequenz bzw. der Gensequenzen erhöht, welche für das Protein kodieren, einen modifizierten Promotor verwendet, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Protein kodiert und gegebenenfalls diese Maßnahmen kombiniert. Zellen mit solcher Genausstattung werden beispielsweise durch Transformation, Transduktion, Konjugation oder eine Kombination dieser Methoden mit einem Vektor erzeugt, der das gewünschte Gen, ein Allel dieses Gens oder Teile davon und einen die Expression des Gens ermöglichenden Promotor enthält. Die heterologe Expression wird insbesondere durch Integration des Gens oder der Allele in das Chromosom der Zelle oder einen extrachromosomal replizierenden Vektor ermöglicht. Einen Überblick über die Möglichkeiten zur rekombinanten Produktion in Zellen am Beispiel der Isocitratlyase gibt EP0839211, die hiermit als Referenz eingeführt wird und deren Offenbarungsgehalt hinsichtlich der Möglichkeiten zur rekombinanten Produktion in Zellen einen Teil der Offenbarung der vorliegenden Erfindung bildet.

Die Bereitstellung bzw. Produktion bzw. Expression der vorstehend und aller nachfolgend genannten Proteine bzw. Gene ist mit Hilfe von 1- und 2-dimensionaler Gelelektrophorese und anschließender optischer Identifizierung der Proteinkonzentration mit entsprechender Auswertesoftware im Gel nachweisbar. Wenn die gefundene Expressionsleistung ausschließlich auf einer Erhöhung der Expression des entsprechenden Gens basiert, so kann die Quantifizierung der rekombinante Expression in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden.

Wird die rekombinante Expression durch Erhöhung der Synthese eines Proteins bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. Durch induzierbare Promotoren ist es zusätzlich möglich, die Expression zu jedem beliebigen Zeitpunkt zu steigern. Des Weiteren können dem Protein-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert.

Zur Erhöhung der rekombinante Expression der jeweiligen Gene werden zum Beispiel episomale Plasmide eingesetzt. Als Plasmide bzw. Vektoren kommen im Prinzip alle dem Fachmann für diesen Zweck zur Verfügung stehenden Ausführungsformen in Frage. Derartige Plasmide und Vektoren können z. B. den Broschüren der Firmen Novagen, Promega, New England Biolabs, Clontech oder Gibco BRL entnommen werden. Weitere bevorzugte Plasmide und Vektoren können gefunden werden in: Glover, D. M. (1985), DNA cloning: a practical approach, Vol. I-III, IRL Press Ltd. , Oxford; Rodriguez, R.L. und Denhardt, D. T (eds) (1988), Vectors : a survey of molecular cloning vectors and their uses, 179-204, Butterworth, Stoneham; Goeddel, D. V. (1990), Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbour Laboratory Press, New York.

Der Plasmidvektor, der das zu amplifizierende Gen enthält, wird anschließend durch Konjugation oder Transformation in den gewünschten Stamm überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Letters 123: 343-347 (1994) beschrieben. Nach homologer Rekombination mittels eines "cross-over"-Ereignisses enthält der resultierende Stamm mindestens zwei Kopien des betreffenden Gens.

Es ist daher in dem erfindungsgemäßen Verfahren bevorzugt, dass rekombinante Mikroorganismen eingesetzt werden, aufgrund der guten genetischen Zugänglichkeit ist der Mikroorganismus bevorzugt ausgewählt aus der Gruppe der Bakterien, insbesondere der gram-negativen, besonders aus der Gruppe enthaltend, bevorzugt bestehend aus, *E. coli, Pseudomonas sp., Pseudomonas fluorescens, Pseudomonas putida*, *Pseudomonas acidovorans, Pseudomonas aeruginosa, Acidovorax sp., Acidovorax temperans, Acinetobacter* sp., *Burkholderia* sp., Cyanobakterien, *Klebsiella* sp., *Salmonella sp., Rhizobium sp.* und *Rhizobium meliloti,* wobei *E. coli* besonders bevorzugt ist.

Die in dem erfindungsgemäßen Verfahren eingesetzten Zellen sind ebenso Bestandteil der vorliegenden Erfindung.

Somit sind Mikroorganismen, die derart gentechnisch verändert wurden, dass sie mindestens ein eine organische Substanz oxidierendes Enzym und mindestens ein alkL-Genprodukt verstärkt synthetisieren, wobei das alkL-Genprodukt unabhängig von mindestens einem weiteren, durch das das alkL-Gen enthaltende alk-Operon kodierten Genprodukt, welches in natürlich vorkommender Form an das Entstehen des alkL-Genproduktes gekoppelt ist, synthetisiert wird, dass das weitere Genprodukt ausgewählt ist aus mindestens einem aus der Gruppe bestehend aus AlkF, AlkG, und AlkH und dass das alkL-Genprodukt ausgewählt ist aus der Gruppe bestehend aus Proteine kodiert durch die alkL-Gene aus *Pseudomonas putida* GPO1 und P1, welche wiedergegebenen werden durch Seq ID Nr. 1 und Seq ID Nr. 3 und Proteine mit Polypeptidsequenz Seq ID Nr. 2 oder Seq ID Nr. 4,Gegenstand der vorliegenden Erfindung.

Bevorzugte oxidierende Enzyme sind in diesem Zusammenhang dieselben oxidierenden Enzyme, die in dem erfindungsgemäßen Verfahren bevorzugt eingesetzt werden; analoges gilt für bevorzugte alkL-Genprodukte, bevorzugte durch das das alkL-Gen enthaltende alk-Operon kodierte Genprodukte, bevorzugte organische Substanzen, sowie bevorzugte Mikroorganismen.

Noch ein Gegenstand der vorliegenden Erfindung ist die Verwendung eines alkL-Genproduktes, bevorzugt in einem Mikroorganismus, zur Erhöhung der Oxidationsrate mindestens eines eine organische Substanz oxidierenden Enzyms, dadurch gekennzeichnet, dass die Verwendung des alkL-Genproduktes unabhängig von mindestens einem weiteren, durch das das alkL-Gen enthaltende alk-Operon kodierten Genprodukt, welches in natürlich vorkommender Form an das Entstehen des alkL-Genproduktes gekoppelt ist, erfolgt, dass das weitere Genprodukt ausgewählt ist aus mindestens einem aus der Gruppe bestehend aus AlkF, AlkG, und AlkH, und dass das alkL-Genprodukt ausgewählt ist aus der Gruppe bestehend aus Proteine kodiert durch die alkL-Gene aus *Pseudomonas putida* GPO1 und P1, welche wiedergegebenen werden durch Seq ID Nr. 1 und Seq ID Nr. 3 und Proteine mit Polypeptidsequenz Seq ID Nr. 2 oder Seq ID Nr. 4, und dass das oxidierende Enzym und das alkL-Genprodukt in einem Mikroorganismus rekombinant synthetisiert werden.

In diesem Zusammenhang handelt es sich bei der Oxidation bevorzugt um die Oxidation einer organischen Substanz zu einem Aldehyd oder einem Alkohol, insbesondere zu einem Alkohol. Somit wird in diesem Zusammenhang bevorzugt die Hydroxylierungsrate erhöht, insbesondere in $\omega$-Position bei Carbonsäuren, bevorzugt in Bezug auf die Umsetzung von Carbonsäuren und deren Estern zu den entsprechenden $\omega$-hydroxylierten Verbindungen, insbesondere Dodecansäuremethylester zu Hydroxydodecansäuremethylester, Bevorzugt oxidierende Enzyme sind in diesem Zusammenhang dieselben oxidierenden Enzyme, die in dem erfindungsgemäßen Verfahren bevorzugt eingesetzt werden; analoges gilt für bevorzugte alkL-Genprodukte, bevorzugte durch das das alkL-Gen enthaltende alk-Operon kodierte Genprodukte, bevorzugte organische Substanzen und bevorzugte Mikroorganismen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Figuren sind Bestandteil der Beispiele:
Figur 1: *E. coli* Plasmid "pBT10_alkL"

Beispiele:

*Vergleichsbeispiel 1: Expressionsvektor für das AlkBGT Alkanhydroxylase-System aus Pseudomonas putida GPo1 ohne alkL*

Ausgehend von den pCOM Systemen (Smits et al., 2001 Plasmid 64:16-24) wurde das Konstrukt pBT10 (Seq ID Nr. 5) hergestellt, das die drei Komponenten Alkan-Hydroxylase (AlkB), Rubredoxin (AlkG) und Rubredoxin-Reduktase (AlkT) aus *Pseudomonas putida* enthält. Für die Expression der drei Gene wurde die alkBFG Gensequenz unter die Kontrolle des alkB-Promotors gestellt und das alkT-Gen unter die des alkS-Promotors.

Zur Vereinfachung der Klonierung von *alkB* und *alkG* wurde das dazwischen liegende Gen *alkF* gemeinsam mit *alkB* und *alkG* amplifiziert und kloniert. AlkF ist für die zu katalysierende Reaktion ohne Bedeutung.

Eine detaillierte Beschreibung der Herstellung des Vektors pBT10 ist der WO2009077461 zu entnehmen.

*Beispiel 1: Expressionsvektor für das AlkBGT Alkanhydroxylase-System aus Pseudomonas putida GPo1 mit alkL*

In einem weiteren Ansatz wurde gezielt das alkL-Gen in das alkBFG-Operon kloniert, um es zusammen mit dem zur Oxidation nötigen Minimalsatz an Enzymen synthetisieren zu können.

Dazu wurde das alkL-Gen vom pGEc47 (Eggink et al., 1987, J Biol Chem 262, 17712-17718) per PCR amplifiziert.

Die dazu verwendeten Primer P1 und P2 enthalten zur Klonierung in die SalI Schnittstelle des Plasmids pBT10 außerhalb der Zielsequenz ebenfalls SalI Schnittstellen. In den forward-primer P1 wurde darüber hinaus ein Stop-Codon hinter der SalI Schnittstelle eingebaut, um eine mögliche Translation der alkH Reste zu beenden.

P1 ACGCGTCGACCTGTAACGACAACAAAACGAGGGTAG (Seq ID Nr. 6)

P2 ACGCGTCGACCTGCGACAGTGACAGACCTG (Seq ID Nr. 7)

Zur Amplifiktation wurde die Finnzyme Phusion Polymerase (New England Biolabs) verwendet.

Nach Herstellervorschrift wurden 34 μL H2O, 10 μL 5x Phusion HF Puffer, 1 μL dNTPs (10 mM each), 1,25 μL P1, 1,25 μL P2 (für eine End-Primer-Konzentration von 0,5 μM), 2μL pGEc47 Plasmidlösung (150 ng/μL) und 0,5 μL Phusion Polymerase gemischt und in dünnwandigen PCR-Eppendorf-Gefäßen zur PCR verwendet.

Folgendes PCR Programm wurde nach Vorschlag des Polymeraseherstellers programmiert:

[98°C / 30 sec], ([98°C / 10 sec] [72°C / 60 sec]) 30x, [72°C / 10 min]

Das entstandene PCR-Produkt mit 754 bp Länge wurde mit Hilfe des "peqGOLD cycle pure Kits" (PEQLAB Biotechnology GmbH, Erlangen) nach Herstellervorschirft aufgereinigt und mit T4 Polynucleotidkinase phosphoryliert.

Dazu wurden 15 µL der aus der Aufreinigung erhaltenen PCR-Produkt-Lösung mit 2 µL ATP-Lösung (100 mM), 2 µL Kinase-Puffer und 1 µL T4 Polynukleotidkinase gemischt und für 20 Minuten bei 37°C inkubiert. Anschließend wurde das Enzym durch Erhitzen auf 75°C für 10 Minuten abgetötet.

Das so vorbereitete PCR-Produkt wurde nun nach Herstellervorschrift in den pSMART Vektor der Firma lucigen ligiert. 2µL des Ligationsansatzes wurden durch Hitzeschock (42°C für 45sec) in chemisch kompetente DH5α E. coli Zellen transformiert.

Nach Über-Nacht-Inkubation auf Kanamycin-Platten selektionierte Kolonien wurden in Flüssigkultur (5 mL LB Medium mit 30 µg/mL Kanamycin) über Nacht bei 37°C angezüchtet und die Plasmide mit Hilfe des peqGOLD Miniprep Kits (PEQLAB Biotechnologie GmbH (Erlangen)) isoliert.

Durch Restriktionsspaltung mit SalI und anschließender Gelelektrophorese wurden korrekt ligierte Plasmide identifiziert.

Ein solches Plasmid wurde in größerer Menge präpariert und mit SalI gespalten. Das entstehende 693 bp Fragment wurde durch Aufreinigung aus dem Agarosegel (peqGOLD Gel Extraction Kit) isoliert.

Das Plasmid pBT10 wurde ebenfalls in größerer Menge präpariert, mit SalI gespalten und die Enden mit Alkalischer Phosphatase (calf intestin [alkaline] phosphatase, CIP)(NEB) dephosphoryliert.

Diese Prozeduren wurden gleichzeitig in einem Reaktionsgefäß durchgeführt. Dazu wurden 13,3 µL Plasmid-DNA mit 4 µL Puffer, 19,2 µL Wasser, 2 µL Alkalische Phosphatase und 1,5 µL SalI (NEB) gemischt und für 2h bei 37°C inkubiert. Der geschnittene und dephosphorylierte Vektor wurde ebenfalls wie oben beschrieben über ein Agarosegel aufgereinigt.

Zur Einstellung der korrekten Verhältnisse von Vektor und Insert in der Ligation wurden die Konzentrationen der entsprechenden DNA-Lösungen per Agarose-Gelelektrophorese festgestellt.

Zur Ligation wurden 10 µL geschnittener Vektor-DNA-Lösung mit 5 µL Insert-DNA-Lösung gemischt so dass das DNA-Massenverhältnis 1:5 betrug, mit 2 µL Ligasepuffer, 1 µL Wasser sowie 1 µL Ligase versetzt, anschließend 2h bei 22°C und danach über Nacht bei 4°C inkubiert. 5 µL dieses Ansatzes wurden per Elektroporation in DH5α *E. coli* Zellen transformiert.

Kanamycinresistente Kolonien wurden in 5 mL LB-Medium mit Antibiotikum über Nacht angezüchtet und die Plasmide wie oben beschrieben präpariert.

Restriktionsspaltung der Plasmid-DNA aus 5 Klonen durch EcoRV zeigte in drei Fällen jeweils Banden bei 8248Bp, 2234Bp und 1266Bp. Dieses Muster bestätigt die korrekte Einklonierung von alkL.

Das erhaltene Plasmid wurde pBT10_alkL (siehe Figur 1) genannt und weist Seq ID Nr. 8 auf.

*Beispiel 2: Umsetzung von Laurinsäuremethylester zu ω-Hydroxylaurinsäuremethylester*

Für die Biotransformation wurden die Plasmide pBT10 oder pBT10_alkL durch Hitzschock bei 42°C für 2 min in den chemisch kompetenten Stamm *E. coli* W3110 transformiert (Hanahan D, DNA cloning: A practival approach. IRL Press, Oxford, 109-135). Für die Synthese von Hydroxylaurinsäure-methylester wurden *E. coli* W3110-pBT10 und W3110-pBT10_alkL über Nacht bei 30°C und 180 rpm in 100 mL M9 Medium (Na2HPO4 6g/L, KH2PO4 3 g/L, NaCl 0.5 g/L, NH4Cl 1 g/L, 2 mM MgSO4, 0,1 mM CaCl2, 0,5 % Glukose) mit 30 mg/L Kanamycin kultiviert und durch Zentrifugation geerntet. Ein Teil der Biomasse wurde steril in 250 mL M9 Medium mit 0,5 % Glucose und 30 mg/L Kanamycin zu einer OD450 = 0,2 resuspendiert und im Schüttelkolben bei 30°C und 180 rpm weiter kultiviert. Die Expression der alk-Gene wurde nach einer Wachstumszeit von 4 h durch Zugabe von 0,025% (v/v) Dicyclopropylketon induziert und die Kultur unter den gleichen Bedingungen für 4 weitere Stunden geschüttelt. Die Zellen wurden anschließend abzentrifugiert, das Zellpellet in KPi-Puffer (50 mM, pH 7,4) resuspendiert und in einen auf 30°C temperierten Bioreaktor gegeben. Es wurde eine Biomassekonzentration von etwa 1,8 g CDW/L eingestellt. Unter kräftigem Rühren (1500 min-1) und einem Luftzufluss von 2 vvm (Volumen pro Volumen und Minute) wurde das Substrat Laurinsäure-Methylester im Verhältnis 1:2 zur Zellsuspension zugesetzt (100 ml Zellsuspension, 50 ml Laurinsäure-Methylester). Die Temperatur wurde bei 30°C konstant gehalten.

Die Bildung des Hydroxylaurinsäure-Methylesters wurde durch GC-Analyse des Reaktionsansatzes nachgewiesen. Dazu wurde nach 0 min als Negativkontrolle und nach 150 min mit einer Spritze eine Probe durch das Steigrohr des Reaktors genommen und in einem 2 mL Eppendorf Gefäß in einer Eppendorf Tischzentrifuge bei 13200 rpm zur Phasentrennung 5 Minuten lang zentrifugiert. Die organische Phase wurde mittels Gaschromatographie (Thermo Trace GC Ultra) analysiert. Als Säule diente eine Varian Inc. FactorFourTM VF-5m, Länge: 30 m, Filmdicke: 0,25 µm, Innendurchmesser: 0,25 mm.

Analysebedingungen:

| | |
|---|---|
| Ofentemperatur | 80 - 280 °C |
| Rampe | 15°C/min |
| Splitratio | 15 |
| Injektionsvolumen | 1 µl |
| Carrierflow | 1,5 ml/min |
| PTV Injektor | 80 - 280°C bei 15°C/s |
| Detektorbasistemperatur: | 320 °C |

Die gemessenen Bildungsraten für 12-Hydroxylaurinsäuremethylester können nun auf die Aktivität des Biokatalysators umgerechnet und auf die eingesetzte Zellmasse bezogen werden.

Im linearen Bereich der Umsatzkinetik gilt für die Aktivität

Aktivität [U] = umgesetzte Stoffmenge [µmol] / Zeit [min]

Diese zur Enzymbeschreibung übliche Einheit "U" ist ein Maß für die Leistungsfähigkeit eines solchen Biokatalysators am Anfang der Reaktion.

| Stamm | Anfangsaktivität [U/$g_{CDW}$] |
|---|---|
| W3110 pBT10 | 1, 82 |
| W3110 pBT10_alkL | 48,6 |

Die Anfangsaktivität konnte durch das zusätzlich exprimierte alkL um den Faktor 26,7 gesteigert werden.

## Patentansprüche

1. Verfahren zur Oxidation einer organischen Substanz unter Einsatz mindestens eines oxidierenden Enzyms und mindestens eines alkL-Genproduktes, **dadurch gekennzeichnet, dass** das alkL-Genprodukt unabhängig von mindestens einem weiteren, durch das das alkL-Gen enthaltende alk-Operon kodierten Genprodukt, welches in natürlich vorkommender Form an das Entstehen des alkL-Genproduktes gekoppelt ist, bereitgestellt wird,
dass das weitere Genprodukt ausgewählt ist aus mindestens einem aus der Gruppe bestehend aus AlkF, AlkG, und AlkH, und
dass das alkL-Genprodukt ausgewählt ist aus der Gruppe bestehend aus
Proteine kodiert durch die alkL-Gene aus *Pseudomonas putida* GPO1 und P1, welche wiedergegebenen werden durch Seq ID Nr. 1 und Seq ID Nr. 3 und
Proteine mit Polypeptidsequenz Seq ID Nr. 2 oder Seq ID Nr. 4.

2. Verfahren gemäß Anspruch 1, **dadurch gekennzeichnet, dass** die organische Substanz ausgewählt ist aus der Gruppe enthaltend,
verzweigte oder unverzweigte, gesättigte oder ungesättigte, gegebenenfalls substituierte Alkane, Alkene, Alkine, Alkohole, Aldehyde, Ketone, Carbonsäuren, Carbonsäureester, Amine und Epoxide, wobei diese bevorzugt 3 bis 22, insbesondere 6 bis 18, weiterhin bevorzugt 8 bis 14, insbesondere 12 Kohlenstoffatome aufweisen.

3. Verfahren gemäß Anspruch 1 oder 2, **dadurch gekennzeichnet, dass** die organische Substanz ausgewählt ist aus der Gruppe enthaltend,
Carbonsäuren und deren korrespondierenden Ester, unsubstituierte Alkane mit 3 bis 22 Kohlenstoffatomen, unsubstituierte Alkene mit 3 bis 22 Kohlenstoffatomen, unsubstituierte, einwertige Alkohole mit 3 bis 22 Kohlenstoffatomen, unsubstituierte Aldehyde mit 3 bis 22 Kohlenstoffatomen unsubstituierte, einwertige Amine mit 3 bis 22 Kohlenstoffatomen
sowie substituierte Verbindungen, die insbesondere als weitere Substituenten einen oder mehrer Hydroxy-, Amin-, Keto-, Carboxyl-, Cyclopropylreste oder Epoxy-Funktionen tragen.

**4.** Verfahren gemäß mindestens einem der Ansprüche 1 bis 3, **dadurch gekennzeichnet, dass** die organische Substanz zu einem Alkohol, zu einem Aldehyd, zu einem Keton oder zu einer Säure oxidiert wird.

**5.** Verfahren gemäß mindestens einem der Ansprüche 1 bis 4, **dadurch gekennzeichnet, dass** die organische Substanz, insbesondere eine Carbonsäure oder ein Carbonsäureester, an der $\omega$-Position oxidiert wird.

**6.** Verfahren gemäß mindestens einem der Ansprüche 1 bis 5, **dadurch gekennzeichnet, dass** das oxidierende Enzym eine Alkanmonooxygenase, eine Xylolmonooxygenase, eine Aldehyddehydrogenase, eine Alkoholoxidase oder eine Alkoholdehydrogenase, vorzugsweise eine Alkanmonooxygenase ist.

**7.** Verfahren gemäß Anspruch 6, **dadurch gekennzeichnet, dass** die Alkanmonooxygenase eine Cytochrom-P450-Monoxygenase, insbesondere eine Cytochrom-P450-Monoxygenase aus *Candida,* beispielsweise aus *Candida tropicalis,* oder aus Pflanzen, beispielsweise aus *Cicer arietinum L.* ist.

**8.** Verfahren gemäß Anspruch 6, **dadurch gekennzeichnet, dass** die Alkanmonooxygenase ein alkB-Genprodukt ist, welches kodiert wird von einem alkB-Gen aus Organismen ausgewählt aus der Gruppe der gram-negativen Bakterien, insbesondere aus der Gruppe der Pseudomonaden, insbesondere *Pseudomonas putida* GPo1.

**9.** Verfahren gemäß Anspruch 6, **dadurch gekennzeichnet, dass** die Alkoholdehydrogenase die vom *alk*J-Gen kodierte Alkoholdehydrogenase, insbesondere die vom *alk*J-Gen kodierte Alkoholdehydrogenase aus der Gruppe der gram-negativen Bakterien, insbesondere aus der Gruppe der Pseudomonaden, insbesondere aus *Pseudomonas putida* GPo1 ist.

**10.** Verfahren gemäß mindestens einem der Ansprüche 1 bis 9, **dadurch gekennzeichnet, dass** es in mindestens einem Mikroorganismus oder in einem den mindestens einen Mikroorganismus umgebenden Medium durchgeführt wird.

**11.** Verfahren gemäß Anspruch 10, **dadurch gekennzeichnet, dass** der Mikroorganismus ausgewählt ist aus der Gruppe der Bakterien, insbesondere der gram-negativen, besonders aus der Gruppe enthaltend *E. coli, Pseudomonas sp., Pseudomonas fluorescens, Pseudomonas putida, Pseudomonas acidovorans, Pseudomonas aeruginosa, Acidovorax sp., Acidovorax temperans, Acinetobacter* sp., *Burkholderia* sp., Cyanobakterien, *Klebsiella* sp., *Salmonella sp., Rhizobium sp.* und *Rhizobium meliloti.*

**12.** Mikroorganismus, der derart gentechnisch verändert wurde, dass er mindestens ein eine organische Substanz oxidierendes Enzym und mindestens ein alkL-Genprodukt verstärkt synthetisiert, **dadurch gekennzeichnet, dass** das alkL-Genprodukt unabhängig von mindestens einem weiteren, durch das das alkL-Gen enthaltende alk-Operon kodierten Genprodukt, welches in natürlich vorkommender Form an das Entstehen des alkL-Genproduktes gekoppelt ist, synthetisiert wird,
dass das weitere Genprodukt ausgewählt ist aus mindestens einem aus der Gruppe bestehend aus AlkF, AlkG, und AlkH und
dass das alkL-Genprodukt ausgewählt ist aus der Gruppe bestehend aus
Proteine kodiert durch die alkL-Gene aus *Pseudomonas putida* GPO1 und P1, welche wiedergegebenen werden durch Seq ID Nr. 1 und Seq ID Nr. 3 und
Proteine mit Polypeptidsequenz Seq ID Nr. 2 oder Seq ID Nr. 4.

**13.** Verwendung eines alkL-Genproduktes zur Erhöhung der Oxidationsrate mindestens eines eine organische Substanz oxidierenden Enzyms, **dadurch gekennzeichnet dass** die Verwendung des alkL-Genproduktes unabhängig von mindestens einem weiteren, durch das das alkL-Gen enthaltende alk-Operon kodierten Genprodukt, welches in natürlich vorkommender Form an das Entstehen des alkL-Genproduktes gekoppelt ist, erfolgt,
dass das weitere Genprodukt ausgewählt ist aus mindestens einem aus der Gruppe bestehend aus AlkF, AlkG, und AlkH , und
dass das alkL-Genprodukt ausgewählt ist aus der Gruppe bestehend aus
Proteine kodiert durch die alkL-Gene aus *Pseudomonas putida* GPO1 und P1, welche wiedergegebenen werden durch Seq ID Nr. 1 und Seq ID Nr. 3 und
Proteine mit Polypeptidsequenz Seq ID Nr. 2 oder Seq ID Nr. 4, und
dass das oxidierende Enzym und das alkL-Genprodukt in einem Mikroorganismus rekombinant synthetisiert werden.

## Claims

1. Method for oxidizing an organic substance using at least one oxidizing enzyme and at least one alkL gene product, **characterized in that** the alkL gene product is provided independently of at least one other gene product that is encoded by the alk operon containing the alkL gene and that in a naturally occurring form is coupled to the formation of the alkL gene product, **in that** the further gene product is selected from at least one of the group consisting of AlkF, AlkG and AlkH, and
**in that** the alkL gene product is selected from the group consisting of
proteins encoded by the alkL genes from *Pseudomonas putida* GPo1 and P1, which are given by Seq ID No. 1 and Seq ID No. 3, and
proteins having the polypeptide sequence Seq ID No. 2 or Seq ID No. 4.

2. Method according to Claim 1, **characterized in that** the organic substance is selected from the group consisting of branched or unbranched, saturated or unsaturated, optionally substituted alkanes, alkenes, alkynes, alcohols, aldehydes, ketones, carboxylic acids, esters of carboxylic acids, amines and epoxides, wherein these have preferably 3 to 22, in particular 6 to 18, more preferably 8 to 14, in particular 12, carbon atoms.

3. Method according to Claim 1 or 2, **characterized in that** the organic substance is selected from the group consisting of carboxylic acids and corresponding esters thereof, unsubstituted alkanes having 3 to 22 carbon atoms, unsubstituted alkenes having 3 to 22 carbon atoms, unsubstituted monohydric alcohols having 3 to 22 carbon atoms, unsubstituted aldehydes having 3 to 22 carbon atoms, unsubstituted monobasic amines having 3 to 22 carbon atoms and also substituted compounds that, in particular, as further substituents, carry one or more hydroxyl, amino, keto, carboxyl, cyclopropyl radicals or epoxy functions.

4. Method according to at least one of Claims 1 to 3, **characterized in that** the organic substance is oxidized to an alcohol, to an aldehyde, to a ketone, or to an acid.

5. Method according to at least one of Claims 1 to 4, **characterized in that** the organic substance, in particular a carboxylic acid or an ester of a carboxylic acid, is oxidized at the $\omega$-position.

6. Method according to at least one of Claims 1 to 5, **characterized in that** the oxidizing enzyme is an alkane monooxygenase, a xylene monooxygenase, an aldehyde dehydrogenase, an alcohol oxidase or an alcohol dehydrogenase, preferably an alkane monooxygenase.

7. Method according to Claim 6, **characterized in that** the alkane monooxygenase is a cytochrome-P450 monooxygenase, in particular a cytochrome-P450 monooxygenase from *Candida,* for example from *Candida tropicalis,* or from plants, for example from *Cicer arietinum L.*

8. Method according to Claim 6, **characterized in that** the alkane monooxygenase is an alkB gene product which is encoded by an alkB gene from organisms selected from the group of the Gram-negative bacteria, in particular from the group of the Pseudomonads, in particular *Pseudomonas putida* GPo1.

9. Method according to Claim 6, **characterized in that** the alcohol dehydrogenase is the alcohol dehydrogenase encoded by the *alk*J gene, in particular the alcohol dehydrogenase encoded by the *alk*J gene from the group of the Gram-negative bacteria, in particular from the group of the Pseudomonads, in particular from *Pseudomonas putida* GPo1.

10. Method according to at least one of Claims 1 to 9, **characterized in that** it is carried out in at least one microorganism or in a medium surrounding the at least one microorganism.

11. Method according to Claim 10, **characterized in that** the microorganism is selected from the group of the bacteria, in particular the Gram-negative, in particular from the group containing *E. coli, Pseudomonas sp., Pseudomonas fluorescens, Pseudomonas putida, Pseudomonas acidovorans, Pseudomonas aeruginosa, Acidovorax sp., Aci-*

*dovorax temperans, Acinetobacter* sp., *Burkholderia* sp., cyanobacteria, *Klebsiella* sp., *Salmonella sp., Rhizobium sp.* and *Rhizobium meliloti.*

**12.** Microorganism which has been genetically modified in such a manner that it synthesizes at least one enzyme oxidizing an organic substance and at least one alkL gene product in an amplified manner, **characterized in that** the alkL gene product is synthesized independently of at least one other gene product that is encoded by the alk operon containing the alkL gene and that in a naturally occurring form is coupled to the formation of the alkL gene product, **in that** the further gene product is selected from at least one of the group consisting of
AlkF, AlkG and AlkH, and
**in that** the alkL gene product is selected from the group consisting of
proteins encoded by the alkL genes from *Pseudomonas putida* GPo1 and P1, which are given by Seq ID No. 1 and Seq ID No. 3, and
proteins having the polypeptide sequence Seq ID No. 2 or Seq ID No. 4.

**13.** Use of an alkL gene product for increasing the oxidation rate of at least one enzyme oxidizing an organic substance, **characterized in that** the use of the alkL gene product proceeds independently of at least one other gene product that is encoded by the alk operon containing the alkL gene and that in a naturally occurring form is coupled to the formation of the alkL gene product, **in that** the further gene product is selected from at least one of the group consisting of AlkF, AlkG and AlkH, and
**in that** the alkL gene product is selected from the group consisting of
proteins encoded by the alkL genes from *Pseudomonas putida* GPo1 and P1, which are given by Seq ID No. 1 and Seq ID No. 3, and
proteins having the polypeptide sequence Seq ID No. 2 or Seq ID No. 4, and **in that** the oxidizing enzyme and the alkL gene product are synthesized recombinantly in a microorganism.

**Revendications**

**1.** Procédé d'oxydation d'une substance organique en utilisant au moins une enzyme oxydante et au moins un produit génique alkL, **caractérisé en ce que** le produit génique alkL est préparé indépendamment d'au moins un autre produit génique codé par l'opéron alk contenant le gène alkL, qui est couplé sous forme naturelle à la formation du produit génique alkL,
**en ce que** l'autre produit génique est choisi parmi au moins un du groupe constitué par alkF, alkG et alkH, et **en ce que** le produit génique alkL est choisi dans le groupe constitué par :

les protéines codées par les gènes alkL de *Pseudomonas putida* GPO1 et P1, qui sont représentées par Seq ID n° 1 et Seq ID n° 3, et
les protéines ayant une séquence polypeptidique Seq ID n° 2 ou Seq ID n° 4.

**2.** Procédé selon la revendication 1, **caractérisé en ce que** la substance organique est choisie dans le groupe contenant les alcanes, alcènes, alcynes, alcools, aldéhydes, cétones, acides carboxyliques, esters d'acides carboxyliques, amines et époxydes ramifiés ou non ramifiés, saturés ou insaturés, éventuellement substitués, ceux-ci comprenant de préférence 3 à 22, notamment 6 à 18, de manière davantage préférée 8 à 14, notamment 12 atomes de carbone.

**3.** Procédé selon la revendication 1 ou 2, **caractérisé en ce que** la substance organique est choisie dans le groupe contenant les acides carboxyliques et leurs esters correspondants, les alcanes non substitués de 3 à 22 atomes de carbone, les alcènes non substitués de 3 à 22 atomes de carbone, les alcools monovalents non substitués de 3 à 22 atomes de carbone, les aldéhydes non substitués de 3 à 22 atomes de carbone, les amines monovalentes non substituées de 3 à 22 atomes de carbone, ainsi que les composés substitués, qui portent notamment en tant qu'autres substituants un ou plusieurs radicaux hydroxy, amino, céto, carboxyle, cyclopropyle ou une ou plusieurs fonctions époxy.

**4.** Procédé selon au moins l'une quelconque des revendications 1 à 3, **caractérisé en ce que** la substance organique est oxydée en un alcool, en un aldéhyde, en une cétone ou en un acide.

**5.** Procédé selon au moins l'une quelconque des revendications 1 à 4, **caractérisé en ce que** la substance organique, notamment un acide carboxylique ou un ester d'acide carboxylique, est oxydée en position $\omega$.

6. Procédé selon au moins l'une quelconque des revendications 1 à 5, **caractérisé en ce que** l'enzyme oxydante est une alcane-monooxygénase, une xylène-monooxygénase, une aldéhyde-déshydrogénase, une alcool-oxydase ou une alcool-déshydrogénase, de préférence une alcane-monooxygénase.

7. Procédé selon la revendication 6, **caractérisé en ce que** l'alcane-monooxygénase est une cytochrome-P450-monooxygénase, notamment une cytochrome-P450-monooxygénase issue de *Candida,* par exemple issue de *Candida tropicalis,* ou issue de plantes, par exemple issue de *Cicer arietinum L.*

8. Procédé selon la revendication 6, **caractérisé en ce que** l'alcane-monooxygénase est un produit génique alkB, qui est codé par un gène alkB issu d'organismes choisis dans le groupe constitué par les bactéries à gram négatif, notamment dans le groupe constitué par les Pseudomonas, notamment *Pseudomonas putida* GPo1.

9. Procédé selon la revendication 6, **caractérisé en ce que** l'alcool-déshydrogénase est l'alcool-déshydrogénase codée par le gène *alk*J, notamment l'alcool-déshydrogénase codée par le gène *alk*J issue du groupe des bactéries à gram négatif, notamment issue du groupe des Pseudomonas, notamment issue de *Pseudomonas putida* GPo1.

10. Procédé selon au moins l'une quelconque des revendications 1 à 9, **caractérisé en ce qu'**il est réalisé dans au moins un microorganisme ou dans un milieu entourant ledit au moins un microorganisme.

11. Procédé selon la revendication 10, **caractérisé en ce que** le microorganisme est choisi dans le groupe constitué par les bactéries, notamment à gram négatif, en particulier du groupe contenant *E. coli, Pseudomonas sp., Pseudomonas fluorescens, Pseudomonas putida, Pseudomonas acidovorans, Pseudomonas aeruginosa, Acidovorax sp., Acidovorax temperans, Acinetobacter sp., Burkholderia sp.,* les cyanobactéries, *Klebsiella sp., Salmonella sp., Rhizobium sp. et Rhizobium meliloti.*

12. Microorganisme, qui a été modifié génétiquement de telle sorte qu'il synthétise de manière renforcée au moins une enzyme oxydant une substance organique et au moins un produit génique alkL, **caractérisé en ce que** le produit génique alkL est synthétisé indépendamment d'au moins un autre produit génique codé par l'opéron alk contenant le gène alkL, qui est couplé sous forme naturelle à la formation du produit génique alkL,
**en ce que** l'autre produit génique est choisi parmi au moins un du groupe constitué par AlkF, AlkG et AlkH, et **en ce que** le produit génique alkL est choisi dans le groupe constitué par :

    les protéines codées par les gènes alkL de *Pseudomonas putida* GPO1 et P1, qui sont représentées par Seq ID n° 1 et Seq ID n° 3, et
    les protéines ayant une séquence polypeptidique Seq ID n° 2 ou Seq ID n° 4.

13. Utilisation d'un produit génique alkL pour augmenter le taux d'oxydation d'au moins une enzyme oxydant une substance organique, **caractérisée en ce que** l'utilisation du produit génique alkL a lieu indépendamment d'au moins un autre produit génique codé par l'opéron alk contenant le gène alkL, qui est couplé sous forme naturelle à la formation du produit génique alkL,
**en ce que** l'autre produit génique est choisi parmi au moins un du groupe constitué par alkF, alkG et alkH, et **en ce que** le produit génique alkL est choisi dans le groupe constitué par :

    les protéines codées par les gènes alkL de *Pseudomonas putida* GPO1 et P1, qui sont représentées par Seq ID n° 1 et Seq ID n° 3, et
    les protéines ayant une séquence polypeptidique Seq ID n° 2 ou Seq ID n° 4, et
    **en ce que** l'enzyme oxydante et le produit génique alkL sont synthétisés de manière recombinante dans un microorganisme.

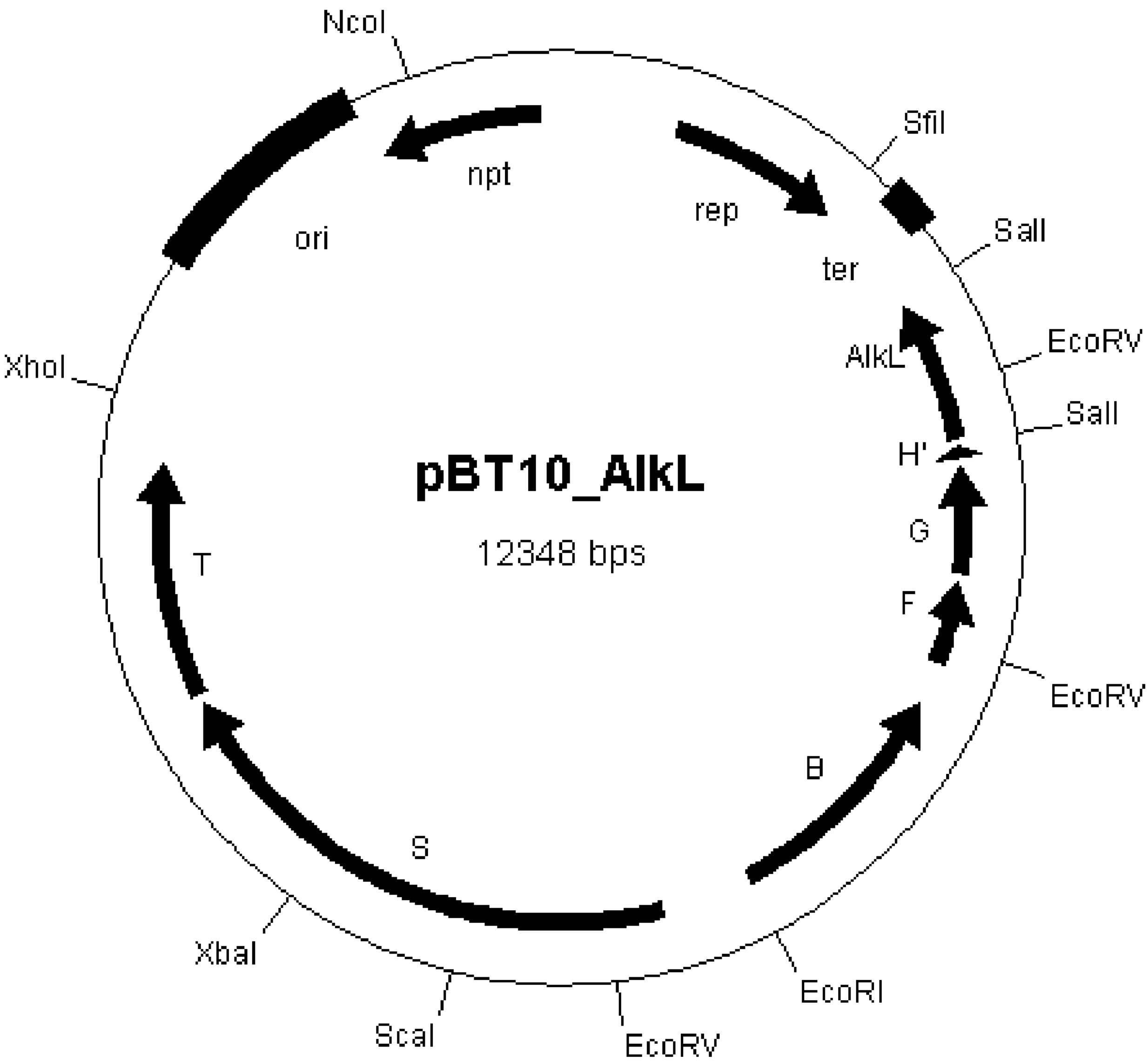
NcoI
SfiI
SalI
EcoRV
SalI
EcoRV
EcoRI
EcoRV
ScaI
XbaI
XhoI
npt
rep
ori
ter
AlkL
H'
G
F
B
S
T
pBT10_AlkL
12348 bps

Figur 1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 277674 A
- WO 2002022845 A
- EP 0502524 A
- US 5306625 A
- WO 0020566 A
- EP 0839211 A
- WO 2009077461 A


### In der Beschreibung aufgeführte Nicht-Patentliteratur


- **CHEN et al.** *J Bacteriol.,* 17. Dezember 1995, vol. 7 (23), 6894-901
- **SCHNEIDER et al.** *Appl Environ Microbiol.,* Oktober 1998, vol. 64 (10), 3784-90
- **FAVRE-BULLE et al.** *Nature Bio/Technology,* April 1991, vol. 9, 367-371
- **ROTHEN et al.** *Biotechnol Bioeng.,* 20. Mai 1998, vol. 58 (4), 356-65
- **CHEN et al.** *J. Bacteriol,* 1995, 6894-6901
- **NIEBOER et al.** *Molecular Microbiology,* 1993, vol. 8 (6), 1039-1051
- **WUBBOLTS et al.** *Biotechnolgy and Bioengineering,* 1996, vol. 52, 301-308
- **BARZ et al.** Cloning and characterization of eight cytochrome P450 cDNAs from chickpea (Cicer arietinum L.) cell suspension cultures. *Plant Science,* 2000, vol. 155, 101-108
- **VAN BEILEN et al.** Functional Analysis of Alkane Hydroxylases from Gram-Negative and Gram-Positive Bacteria. *Journal of Bacteriology,* 2002, vol. 184 (6), 1.733-1.742
- **VAN BEILEN et al.** *Oil & Gas Science and Technology,* 2003, vol. 58 (4), 427-440
- **VAN BEILEN et al.** *Molecular Microbiology,* 1992, vol. 6 (21), 3121-3136
- **HERMANN et al.** *Electrophoresis,* 2001, vol. 22, 1712.23
- **SAMBROOK et al.** Molecular Cloning: a laboratory manual. Cold Spring Harbor Laboratory Press, 1989
- **LOHAUS ; MEYER.** *Biospektrum,* 1989, vol. 5, 32-39
- **LOTTSPEICH.** *Angewandte Chemie,* 1999, vol. 111, 2630-2647
- **GLOVER, D. M.** DNA cloning: a practical approach. IRL Press Ltd, 1985, vol. I-III
- Vectors : a survey of molecular cloning vectors and their uses. Butterworth, 1988, 179-204
- **GOEDDEL, D. V.** Systems for heterologous gene expression. *Methods Enzymol.,* 1990, vol. 185, 3-7
- **SAMBROOK, J. ; FRITSCH, E. F. ; MANIATIS, T.** Molecular cloning: a laboratory manual. Cold Spring Harbour Laboratory Press, 1989
- **SCHÄFER et al.** *Applied and Environmental Microbiology,* 1994, vol. 60, 756-759
- **THIERBACH et al.** *Applied Microbiology and Biotechnology,* 1988, vol. 29, 356-362
- **DUNICAN ; SHIVNAN.** *Bio/Technology,* 1989, vol. 7, 1067-1070
- **TAUCH et al.** *FEMS Microbiology Letters,* 1994, vol. 123, 343-347
- **SMITS et al.** *Plasmid,* 2001, vol. 64, 16-24
- **EGGINK et al.** *J Biol Chem,* 1987, vol. 262, 17712-17718
- **HANAHAN D.** DNA cloning: A practival approach. IRL Press, 109-135

<110> Evonik Degussa GmbH

<120> Biokatalytisches Oxidationsverfahren mit alkL-Genprodukt

<130> 201000056

<160> 8

<170> PatentIn version 3.4

<210> 1
<211> 693
<212> DNA
<213> Pseudomonas putida

<220>
<221> AlkL
<222> (1)..(693)

<220>
<221> CDS
<222> (1)..(693)

<400> 1

```
atg agt ttt tct aat tat aaa gta atc gcg atg ccg gtg ttg gtt gct        48
Met Ser Phe Ser Asn Tyr Lys Val Ile Ala Met Pro Val Leu Val Ala
1               5                   10                  15

aat ttt gtt ttg ggg gcg gcc act gca tgg gcg aat gaa aat tat ccg        96
Asn Phe Val Leu Gly Ala Ala Thr Ala Trp Ala Asn Glu Asn Tyr Pro
            20                  25                  30

gcg aaa tct gct ggc tat aat cag ggt gac tgg gtc gct agc ttc aat       144
Ala Lys Ser Ala Gly Tyr Asn Gln Gly Asp Trp Val Ala Ser Phe Asn
        35                  40                  45

ttt tct aag gtc tat gtg ggt gag gag ctt ggc gat cta aat gtt gga       192
Phe Ser Lys Val Tyr Val Gly Glu Glu Leu Gly Asp Leu Asn Val Gly
    50                  55                  60

ggg ggg gct ttg cca aat gct gat gta agt att ggt aat gat aca aca       240
Gly Gly Ala Leu Pro Asn Ala Asp Val Ser Ile Gly Asn Asp Thr Thr
65                  70                  75                  80

ctt acg ttt gat atc gcc tat ttt gtt agc tca aat ata gcg gtg gat       288
Leu Thr Phe Asp Ile Ala Tyr Phe Val Ser Ser Asn Ile Ala Val Asp
                85                  90                  95

ttt ttt gtt ggg gtg cca gct agg gct aaa ttt caa ggt gag aaa tca       336
Phe Phe Val Gly Val Pro Ala Arg Ala Lys Phe Gln Gly Glu Lys Ser
            100                 105                 110

atc tcc tcg ctg gga aga gtc agt gaa gtt gat tac ggc cct gca att       384
Ile Ser Ser Leu Gly Arg Val Ser Glu Val Asp Tyr Gly Pro Ala Ile
        115                 120                 125

ctt tcg ctt caa tat cat tac gat agc ttt gag cga ctt tat cca tat       432
Leu Ser Leu Gln Tyr His Tyr Asp Ser Phe Glu Arg Leu Tyr Pro Tyr
    130                 135                 140

gtt ggg gtt ggt gtt ggt cgg gtg cta ttt ttt gat aaa acc gac ggt       480
Val Gly Val Gly Val Gly Arg Val Leu Phe Phe Asp Lys Thr Asp Gly


145                 150                 155                 160

gct ttg agt tcg ttt gat att aag gat aaa tgg gcg cct gct ttt cag       528
Ala Leu Ser Ser Phe Asp Ile Lys Asp Lys Trp Ala Pro Ala Phe Gln
                165                 170                 175

gtt ggc ctt aga tat gac ctt ggt aac tca tgg atg cta aat tca gat       576
Val Gly Leu Arg Tyr Asp Leu Gly Asn Ser Trp Met Leu Asn Ser Asp
            180                 185                 190

gtg cgt tat att cct ttc aaa acg gac gtc aca ggt act ctt ggc ccg       624
Val Arg Tyr Ile Pro Phe Lys Thr Asp Val Thr Gly Thr Leu Gly Pro
        195                 200                 205

gtt cct gtt tct act aaa att gag gtt gat cct ttc att ctc agt ctt       672
Val Pro Val Ser Thr Lys Ile Glu Val Asp Pro Phe Ile Leu Ser Leu
    210                 215                 220

ggt gcg tca tat gtt ttc taa                                           693
Gly Ala Ser Tyr Val Phe
225                 230
```

<210> 2
<211> 230
<212> PRT
<213> Pseudomonas putida

<400> 2

```
Met Ser Phe Ser Asn Tyr Lys Val Ile Ala Met Pro Val Leu Val Ala
1               5                   10                  15

Asn Phe Val Leu Gly Ala Ala Thr Ala Trp Ala Asn Glu Asn Tyr Pro
            20                  25                  30

Ala Lys Ser Ala Gly Tyr Asn Gln Gly Asp Trp Val Ala Ser Phe Asn
        35                  40                  45

Phe Ser Lys Val Tyr Val Gly Glu Glu Leu Gly Asp Leu Asn Val Gly
    50                  55                  60

Gly Gly Ala Leu Pro Asn Ala Asp Val Ser Ile Gly Asn Asp Thr Thr
65                  70                  75                  80

Leu Thr Phe Asp Ile Ala Tyr Phe Val Ser Ser Asn Ile Ala Val Asp
                85                  90                  95

Phe Phe Val Gly Val Pro Ala Arg Ala Lys Phe Gln Gly Glu Lys Ser
            100                 105                 110

Ile Ser Ser Leu Gly Arg Val Ser Glu Val Asp Tyr Gly Pro Ala Ile
        115                 120                 125

Leu Ser Leu Gln Tyr His Tyr Asp Ser Phe Glu Arg Leu Tyr Pro Tyr
    130                 135                 140
```

```
Val Gly Val Gly Val Gly Arg Val Leu Phe Phe Asp Lys Thr Asp Gly
145                 150                 155                 160

Ala Leu Ser Ser Phe Asp Ile Lys Asp Lys Trp Ala Pro Ala Phe Gln
                165                 170                 175

Val Gly Leu Arg Tyr Asp Leu Gly Asn Ser Trp Met Leu Asn Ser Asp
            180                 185                 190

Val Arg Tyr Ile Pro Phe Lys Thr Asp Val Thr Gly Thr Leu Gly Pro
        195                 200                 205

Val Pro Val Ser Thr Lys Ile Glu Val Asp Pro Phe Ile Leu Ser Leu
    210                 215                 220

Gly Ala Ser Tyr Val Phe
225                 230
```

<210> 3
<211> 693
<212> DNA
<213> Pseudomonas putida

<220>
<221> AlkL
<222> (1)..(693)

<220>
<221> CDS
<222> (1)..(693)

<400> 3

```
atg aat ccg cct att tta aaa aaa ctc gct atg tcg ata tta gca act      48
Met Asn Pro Pro Ile Leu Lys Lys Leu Ala Met Ser Ile Leu Ala Thr
1               5                   10                  15

agt ttt gtg ttg ggt ggg gcc agt gcg tgg tca ggt gaa atc tat tcg      96
Ser Phe Val Leu Gly Gly Ala Ser Ala Trp Ser Gly Glu Ile Tyr Ser
            20                  25                  30

act gaa act gct ggc tac aat cag ggc gac tgg gtt gct agc ttt aat      144
Thr Glu Thr Ala Gly Tyr Asn Gln Gly Asp Trp Val Ala Ser Phe Asn
        35                  40                  45

atg tct aaa gtt tat gta gac gag acg cta ggc tcc cta aat gta ggt      192
Met Ser Lys Val Tyr Val Asp Glu Thr Leu Gly Ser Leu Asn Val Gly
    50                  55                  60

ggg gct act gta ccc aat gct gct gta agc atc ggt aat gat aca aca      240
Gly Ala Thr Val Pro Asn Ala Ala Val Ser Ile Gly Asn Asp Thr Thr
65                  70                  75                  80

gtt tct ttt gat att tcc tat ttt att agt aac aat gta gct ttg gat      288
Val Ser Phe Asp Ile Ser Tyr Phe Ile Ser Asn Asn Val Ala Leu Asp
                85                  90                  95

ttt ttc gtc ggg att cca gct aaa gct aag ttt caa ggt gaa aaa tcc      336

Phe Phe Val Gly Ile Pro Ala Lys Ala Lys Phe Gln Gly Glu Lys Ser
            100                 105                 110

atc tct gcg ctg gga aga gtc agt gaa gtt gat tat ggc cct gca att      384
Ile Ser Ala Leu Gly Arg Val Ser Glu Val Asp Tyr Gly Pro Ala Ile
        115                 120                 125

ttg tca ctt cag tat cat ttt gat aat ttt gag cga ctt tat cca tat      432
Leu Ser Leu Gln Tyr His Phe Asp Asn Phe Glu Arg Leu Tyr Pro Tyr
    130                 135                 140

gtc gga cta ggt gtc ggt cga gtg ttt ttc ttc gac aaa act gat ggt      480
Val Gly Leu Gly Val Gly Arg Val Phe Phe Phe Asp Lys Thr Asp Gly
145                 150                 155                 160

gcc ttg act tca ttt gat atc aaa gat aaa tgg gcg cct gct gtt cag      528
Ala Leu Thr Ser Phe Asp Ile Lys Asp Lys Trp Ala Pro Ala Val Gln
                165                 170                 175

gtc ggc ctt aga tat gat ttt ggt aac tca tgg atg tta aat tca gat      576
Val Gly Leu Arg Tyr Asp Phe Gly Asn Ser Trp Met Leu Asn Ser Asp
            180                 185                 190

gtg cgc tat att cct ttc aaa aca gat gtt tct ggt aca ctt ggg gct      624
Val Arg Tyr Ile Pro Phe Lys Thr Asp Val Ser Gly Thr Leu Gly Ala
        195                 200                 205

gca cct gtt tct acc aag att gag att gat cct ttc att ctg agt ctt      672
Ala Pro Val Ser Thr Lys Ile Glu Ile Asp Pro Phe Ile Leu Ser Leu
    210                 215                 220

gga gca tca tat aag ttc tga                                          693
Gly Ala Ser Tyr Lys Phe
225                 230
```

<210> 4
<211> 230
<212> PRT
<213> Pseudomonas putida

<400> 4

```
Met Asn Pro Pro Ile Leu Lys Lys Leu Ala Met Ser Ile Leu Ala Thr
1               5                   10                  15

Ser Phe Val Leu Gly Gly Ala Ser Ala Trp Ser Gly Glu Ile Tyr Ser
            20                  25                  30

Thr Glu Thr Ala Gly Tyr Asn Gln Gly Asp Trp Val Ala Ser Phe Asn
        35                  40                  45

Met Ser Lys Val Tyr Val Asp Glu Thr Leu Gly Ser Leu Asn Val Gly
    50                  55                  60

Gly Ala Thr Val Pro Asn Ala Ala Val Ser Ile Gly Asn Asp Thr Thr
65                  70                  75                  80

Val Ser Phe Asp Ile Ser Tyr Phe Ile Ser Asn Asn Val Ala Leu Asp
                85                  90                  95
```

```
Phe Phe Val Gly Ile Pro Ala Lys Ala Lys Phe Gln Gly Glu Lys Ser
            100                 105                 110

Ile Ser Ala Leu Gly Arg Val Ser Glu Val Asp Tyr Gly Pro Ala Ile
        115                 120                 125

Leu Ser Leu Gln Tyr His Phe Asp Asn Phe Glu Arg Leu Tyr Pro Tyr
    130                 135                 140

Val Gly Leu Gly Val Gly Arg Val Phe Phe Phe Asp Lys Thr Asp Gly
145                 150                 155                 160

Ala Leu Thr Ser Phe Asp Ile Lys Asp Lys Trp Ala Pro Ala Val Gln
                165                 170                 175

Val Gly Leu Arg Tyr Asp Phe Gly Asn Ser Trp Met Leu Asn Ser Asp
            180                 185                 190

Val Arg Tyr Ile Pro Phe Lys Thr Asp Val Ser Gly Thr Leu Gly Ala
        195                 200                 205

Ala Pro Val Ser Thr Lys Ile Glu Ile Asp Pro Phe Ile Leu Ser Leu
    210                 215                 220

Gly Ala Ser Tyr Lys Phe
225                 230
```

<210> 5
<211> 11539
<212> DNA
<213> Artificial

<220>
<223> Vector

<400> 5

```
gaaaaccgcc actgcgccgt taccaccgct gcgttcggtc aaggttctgg accagttgcg     60
tgagcgcata cgctacttgc attacagttt acgaaccgaa caggcttatg tcaattcgcc    120
tctcaggcgc cgctggtgcc gctggttgga cgccaagggt gaatccgcct cgataccctg    180
attactcgct tcctgcgccc tctcaggcgg cgatagggga ctggtaaaac ggggattgcc    240
cagacgcctc cccgcccct tcaggggcac aaatgcggcc ccaacggggc cacgtagtgg     300
tgcgtttttt gcgtttccac ccttttcttc cttttccctt ttaaaccttt taggacgtct    360
acaggccacg taatccgtgg cctgtagagt ttaaaaaggg acggatttgt tgccattaag    420
ggacggattt gttgttaaga agggacggat ttgttgttgt aaagggacgg atttgttgta    480
ttgtgggacg cagatacagt gtccccttat acacaaggaa tgtcgaacgt ggcctcaccc    540
```

ccaatggttt acaaaagcaa tgccctggtc gaggccgcgt atcgcctcag tgttcaggaa 600
cagcggatcg ttctggcctg tattagccag gtgaagagga gcgagcctgt caccgatgaa 660
gtgatgtatt cagtgacggc ggaggacata gcgacgatgg cgggtgtccc tatcgaatct 720
tcctacaacc agctcaaaga agcggccctg cgcctgaaac ggcgggaagt ccggttaacc 780
caagagccca atggcaaggg gaaaagaccg agtgtgatga ttaccggctg ggtgcaaaca 840
atcatctacc gggagggtga gggccgtgta gaactcaggt tcaccaaaga catgctgccg 900
tacctgacgg aactcaccaa acagttcacc aaatacgcct tggctgacgt ggccaagatg 960
gacagcaccc acgcgatcag gctttacgag ctgctcatgc aatgggacag catcggccag 1020
cgcgaaatag aaattgacca gctgcgaaag tggtttcaac tggaaggccg gtatccctcg 1080
atcaaggact tcaagttgcg agtgcttgat ccagccgtga cgcagatcaa cgagcacagc 1140
ccgctacagg tggagtgggc gcagcgaaag accgggcgca aggtcacaca tctgttgttc 1200
agttttggac cgaagaagcc cgccaaggcg gtgggtaagg ccccagcgaa gcgcaaggcc 1260
gggaagattt cagatgctga gatcgcgaaa caggctcgcc ctggtgagac atgggaagcg 1320
gcccgcgctc gactaaccca gatgccgctg gatctggcct agaggccgtg gccaccacgg 1380
cccggcctgc ctttcaggct gcattattga agcatttatc agggttattg tctcatgagc 1440
ggatacatat ttgaatgtat ttagaaaaat aaacaaaaga gtttgtagaa acgcaaaaag 1500
gccatccgtc aggatggcct tctgcttaat ttgatgcctg gcagtttatg gcgggcgtcc 1560
tgcccgccac cctccgggcc gttgcttcgc aacgttcaaa tccgctcccg gcggatttgt 1620
cctactcagg agagcgttca ccgacaaaca acagataaaa cgaaaggccc agtctttcga 1680
ctgagccttt cgttttattt gatgcctggc agttccctac tctcgcatgg ggagacccca 1740
cactaccatc ggcgctacgg cgtttcactt ctgagttcgg catggggtca ggtgggacca 1800
ccgcgctact gccgccaggc aaattctgtt ttatcagacc gcttctgcgt tctgatttaa 1860
tctgtatcag gctgaaaatc ttctctcatc cgccaaaaca gaagcttggc tgcaggtcga 1920
caagcgctga atgggtatcg gcactagagt ttaacttggc taggctaatt ggtatggtca 1980
tttttatttt gtcctgaatt acctagaatg acttgaagtt ttaatcttca cttttcctcg 2040
tagagcacat agtcttcttt cgtagccccg caatccggac agcaccagtc atcaggaata 2100
tcctcaaagc gagtacctgg agtaaaaccc tcggcctcat cgcccaacgc ctcatcatat 2160
atatggccac aagtaataca tatccacttc aagtatgctt tcccgccttg ggcatcagac 2220
cttggaggtt gagttttata tagatcttgg cctttaacgt cggcactagg caatttctct 2280
aagcttgtcg gcgcaaccac tgcttcagca gttaaacttg tgccactaac ctcggataaa 2340
tttggcgaag tatgggttga ggtgacgccc ttttcaccta cgccgctctc aattaacatg 2400
aagtcaagct tgtctcgaac ggcgcaatcg ggcagcaccc aatcctcagg aataaggtgc 2460
caaggcgtac ctggagaaaa cccctcatgc acattacccg cactctcatc ataaacataa 2520

ttacaatccg ggcatttata gctagccata ctcatcacca tgtcaaattg ttattttgcg 2580
ttgcggcaaa tttgcgttat ttatttctca tcttcttcct tgtggaagat tttcttacaa 2640
cctgtggggt gctcgcctga tcaagaactt cagtcttagc cgcacagccc tcatttttat 2700
tttcttgatt aagtgggatc actaattctg cggccaaaac tgccattcca gttggagtag 2760
catctgaaat accactttcc gagttggcaa gctgactatt caccccctagc tgtgtttctt 2820
tcgagggaga atccgcgaga aagataaagt ccaccttgtc tcgaactgcg cagtccgggc 2880
atgcccaatc tttggggata tcattccagc tggtgttcgg gtggaaacct tcgtgcggct 2940
ccccttatt ttcatcatag atatactgac aatctggaca ctggtacctt gacattctcc 3000
cactctccta ttaactcagc gttagtcgct gctccgacaa cttcatcaga gtgtaactat 3060
cggagcaggt cgccttcaaa gcaattacag agaggcaatc aaccctcagc actttagcga 3120
agtgcactct ttgtgagagc tttcaacgcc gcacataaaa ttgaagcact tattgtaaat 3180
atcgaccgcc gggctctgcg tgctcacata actacgatgc taccgcagag gtactcgaac 3240
tatgaccagc actactagtg ccaaattttt tcaaataggt ttctcgcatc gaatcatcaa 3300
tttggatctt attaaggtca ccaccagccc aatctactac cttgggatcc ataactgatc 3360
taaaccactg aggaatcatc gccatcaaaa atgcaccagg gtaacccgtc ggaagagccg 3420
gcaggccggg aaaatcccga agtgactgat aagaacgtgt tggatgcgcg tggtgatccg 3480
agtgccgctg aaggtggaac agcactagat tagagacgat gtgattacta ttccaagaat 3540
ggtgcggctt ttgatgctca tatcgaccgt cctccatttt ttgacggagc aagccgtaat 3600
gttcaatata gttcgcactg gtcagctgcc accaaccgaa agccatttga atcggcagga 3660
acaccagcat cttaggtcca aacaaggcaa ggagaacggc gtaaagaata actgtgatga 3720
tcattggttg gaggatttca ttatcgaaac tccaaacgct ttggccacgg cgcgaaaggc 3780
gttgttcctc aagcccccaa gcacgaataa atgctcctgg gatctcacgg attgaaaact 3840
tataaatgct ttctcccatc cgggatgttg caggatccat cggtgtagcg acatcacggt 3900
gatgaccctt attatgctca ataaagaagt gaccgtaccc tacgacagcc aacacaattt 3960
tggccatcca acgatcaaaa gtctccttct tgtgaccgag ttcgtgtcct gtattgagcg 4020
ctagtccgtt cacgataccc agtgacaagg caagcgcacc aatttcaagc caagacattg 4080
gctgagttcc gacccaccat gctgacacaa ttaatgcagc gtaatgcata ggaactgtta 4140
gatatgtcaa aactcgatag taccgctcct tctctagttt cggcaccact tcttcaggcg 4200
gattattaaa gtcctcacca aacatcgcat caagcaatgg aagtgcgccg taccatacga 4260
gcaataccag cccataaaaa atcccccaac cagtttcatt tgcaagccag attccgatca 4320
tcggagtagc cggccacaaa gttgatagta tccagagata tttcttttta tctacgtact 4380
ctggagcgga atccagaact ctgtgtttct caagcatatg gaattctcca atttttatta 4440

aattagtcgc tacgagattt aagacgtaat tttatgccta actgagaaag ttaagccgcc 4500
cactctcact ctcgacatct taaacctgag ctaatcggac gcttgcgcca actacaccta 4560
cgggtagttt ttgctccgtc gtctgctgga aaaacacgag ctggccgcaa gcatgccagg 4620
taccgcgagc tactcgcgac ggctgaaagc accgaaatga gcgagctatc tggtcgattt 4680
tgacccggtg cccgtcttca aaatcggcga aggccgaagt cggccagaaa tagcggccta 4740
cttcagacct tccctagtaa atattttgca ccaccgatca tgccgactac acttaagtgt 4800
agttttaata tttaacaccg taacctatgg tgaaaatttc cagtcagctg gcgcgagaat 4860
agcataatga aaataataat aaataatgat ttcccggtcg ctaaggtcgg agcggatcaa 4920
attacgactc tagtaagtgc caaagttcat agttgcatat atcggccaag attgagtatc 4980
gcggatggag ccgctcccag agtatgcctt tacagagccc cacctggata tgggaaaacc 5040
gttgctcttg cgttcgagtg gctacgccac agaacagccg gacgtcctgc agtgtggctt 5100
tctttaagag ccagttctta cagtgaattt gatatctgcg cagagattat tgagcagctt 5160
gaaactttcg aaatggtaaa attcagccgt gtgagagagg gtgtgagcaa gcctgcgctc 5220
ttgcgagacc ttgcatctag tctttggcag agcacctcga ataacgagat agaaacgcta 5280
gtttgtttgg ataatattaa tcatgactta gacttgccgt tgttgcacgc acttatggag 5340
tttatgttaa atacaccaaa aaatatcagg tttgcagttg caggcaatac aataaaaggg 5400
ttctcgcagc ttaaacttgc aggcgctatg cgggagtaca ccgagaaaga cttggccttt 5460
agcgcagaag aggcggtggc gttagcggag gcagagtctg ttcttggagt tcctgaagaa 5520
cagatagaga ccttggtgca agaagttgag gggtggcctg ctcttgtagt ttttttgtta 5580
aagcgtgagt tgccggccaa gcatatttca gcagtagttg aagtagacaa ttactttagg 5640
gatgaaatat ttgaggcgat tcccgagcgc tatcgtgttt ttcttgcaaa ttcttcattg 5700
ctcgatttcg tgacgcctga tcaatacaat tatgtattca aatgcgtcaa tggggtctca 5760
tgtattaagt atttaagcac taattacatg ttgcttcgcc atgtgagcgg tgagccagcg 5820
cagtttacac tgcatccagt actgcgtaat tttctacgag aaattacttg gactgaaaat 5880
cctgctaaaa gatcctacct gcttaagcgt gcagctttct ggcattggcg tagaggtgaa 5940
taccagtatg caatacgaat atccctacgg gcgaatgact gtcgctgggc agtcagcatg 6000
tctgagagaa taattttaga tttgtcattt cgtcagggcg aaatagatgc gctgagacag 6060
tggctgttag agctgccgaa gcaggcctgg caccaaaaac ccatagtgct tattagttac 6120
gcgtgggtat tgtatttcag tcagcaaggc gcgcgagcag agaagttaat taaagaccta 6180
tcttcacaat ccgataaaaa aaataaatgg caagaaaagg aatggctgca gcttgtgctt 6240
gcaataggta aagcaaccaa agatgaaatg ctttcgagtg aggagctctg taataagtgg 6300
attagtttat ttggggattc aaacgcagtt ggaaaagggg ccgcgctaac ctgtttggct 6360
tttatttttg ccagtgagta tagatttgca gagttggaga aggtgctggc tcaggcccaa 6420

gccgtgaata aatttgcaaa acaaaatttt gcttttggtt ggctgtatgt cgcgaggttt 6480
caacaagccc tagcaagcgg aaaaatgggc tgggcgaggc agattataac tcaagcacgc 6540
acagacagtc gcgcgcagat gatggaatcc gagtttactt cgaaaatgtt tgacgctcta 6600
gagcttgagt tacattatga attgcgctgc ttggacacct cagaagaaaa gctctccaaa 6660
attttagagt tcatttccaa tcacggggtg acagacgtgt ttttttccgt atgccgtgct 6720
gtgtcagctt ggcggcttgg aaggagtgac ctaaatggct ccattgagat attggagtgg 6780
gcgaaggcgc atgcggttga aaaaaatcta ccaagattgg aagttatgag ccaaattgag 6840
atctatcagc gcttagtctg tcaaggcata acgggcataa ataatttaaa aactcttgaa 6900
gatcataaga ttttctccgg acagcactca gccccctaa aagcacgcct gctgcttgtt 6960
caatcactag tgctttcccg agatcggaac tttcatagtg ccgcgcacag agcgttattg 7020
gctattcagc aagcccgtaa aattaacgcg ggccagctgg aagtccgtgg attattgtgt 7080
ttggccggag cgcaggcagg tgccggtgat ttaaaaaagg ctcagcttaa cattgtttat 7140
gcagtggaga tagcaaaaca gcttcaatgc tttcaaacag ttcttgatga agtatgttta 7200
attgagcgaa taataccggc ttcatgtgaa gccttcacag cagttaattt agatcaagcg 7260
attggggctt ttagtcttcc gcgaatagtt gagattggaa agtccgcaga gaataaagct 7320
gacgctttat tgacacggaa gcagattgct gtcttgaggc ttgtaaaaga ggggtgctca 7380
aacaaacaaa tagcaacaaa tatgcatgtc accgaagatg ctataaagtg gcatatgagg 7440
aaaatatttg ccaccttgaa tgtagtgaat cgcacgcaag caacaattga agctgagcgt 7500
caaggaatta tctaaaataa tcggcattaa gtgatatagt gaaaagtata ccggagagag 7560
aattatggca atcgttgttg ttggcgctgg tacagctgga gtaaatgctg cgttctggct 7620
tcgtcaatat ggttataaag ggaaattag gatttttagc agggagtctg tggcgcctta 7680
tcagcggcct cctctatcca aggcttttct gacaagtgag attgcagaat ccgcagtgcc 7740
attaaagcca gaaggttttt atacgaataa caatattacc atttcgttaa atacaccgat 7800
tgtatcaatc gacgtggggc gtaagatagt ttcttctaaa gatggaaaag aatacgcgta 7860
tgaaaaattg attcttgcaa cacctgctag cgcacgtagg ttaacctgcg aggggtctga 7920
actgtctggg gtctgctatt tacgcagtat ggaagacgcc aaaaatttac gtaggaaact 7980
tgtggagagt gcgtctgttg ttgtgttggg cggcggagta atcgggcttg aagtcgcctc 8040
agctgcggtg ggcttaggga agagggtcac agtgatagaa gccacccgc gtgtaatggc 8100
gcgcgtggtt acgccggcag cagcaaactt agtcagagcc cgcctggagg ctgaaggaat 8160
tgagttcaag ctgaatgcga aattaacgtc tataaagggc aggaatggcc atgttgaaca 8220
atgcgtactt gaaagtggag aagaaattca ggcggatctg attgtagttg gaatcggtgc 8280
tatcccagag ctagagctgg caactgaggc ggcccttgaa gtgagtaatg gtgttgtggt 8340

cgatgatcag atgtgtacat cggatacaag tatatatgca atcggcgact gcgcaatggc 8400
tagaaatcct ttttggggaa cgatggtacg tttagagaca attcataatg cggttacaca 8460
cgctcaaatt gtcgcaagta gcatctgtgg cacatcaaca ccagcaccaa ccccaccacg 8520
gttctggtct gatcttaaag ggatggcgct gcaaggactt ggtgctctaa aggactacga 8580
taaactcgtt gttgcaatta ataacgaaac tcttgaacta gaagtccttg cgtacaagca 8640
ggagcgactg attgcaactg agacaataaa tttgcctaaa cgtcaaggtg cgcttgcagg 8700
gagtataaaa ttacctgatt agcaatgatg ctcagccact cgaaccaacg gtcgcgatag 8760
ggacggcagt tacctgccgc cccccgcact ccgtacgtgc ggaactaccg cgtaaaatgt 8820
ggcccaggct gttatgtggc gcttgggcgg ggaagtattg ccatatttgg tgatgaccgt 8880
tttctacgcc acataaatcg gtggtggcta tggtgggatt tcccttgctg aaatgggaga 8940
tccgatcatg ttcgagctct tattcaaata cactgctgtg ttggcggtaa gcgttctcga 9000
gctcatagtc cacgacgccc gtgattttgt agccctggcc gacggccagc aggtaggccg 9060
acaggctcat gccggccgcc gccgcctttt cctcaatcgc tcttcgttcg tctggaaggc 9120
agtacacctt gataggtggg ctgcccttcc tggttggctt ggtttcatca gccatccgct 9180
tgccctcatc tgttacgccg gcggtagccg gccagcctcg cagagcagga ttcccgttga 9240
gcaccgccag gtgcgaataa gggacagtga agaaggaaca cccgctcgcg ggtgggccta 9300
cttcacctat cctgcccggc tgacgccgtt ggatacacca aggaaagtct acacgaaccc 9360
tttggcaaaa tcctgtatat cgtgcgaaaa aggatggata taccgaaaaa atcgctataa 9420
tgaccccgaa gcagggttat gcagcggaaa agcgctgctt ccctgctgtt ttgtggaata 9480
tctaccgact ggaaacaggc aaatgcagga aattactgaa ctgaggggac aggcgagaga 9540
ggatcaatgg ctatctgggg gaccgagggc tgtcgctgcg ccaaggcacg attggagatc 9600
ccctatgcgg tgtgaaatac cgcacagatg cgtaaggaga aaataccgca tcaggcgctc 9660
ttccgcttcc tcgctcactg actcgctgcg ctcggtcgtt cggctgcggc gagcggtatc 9720
agctcactca aaggcggtaa tacggttatc cacagaatca ggggataacg caggaaagaa 9780
catgtgagca aaaggccagc aaaaggccag gaaccgtaaa aaggccgcgt tgctggcgtt 9840
tttccatagg ctccgccccc ctgacgagca tcacaaaaat cgacgctcaa gtcagaggtg 9900
gcgaaacccg acaggactat aaagatacca ggcgtttccc cctggaagct ccctcgtgcg 9960
ctctcctgtt ccgaccctgc cgcttaccgg atacctgtcc gcctttctcc cttcgggaag 10020
cgtggcgctt tctcatagct cacgctgtag gtatctcagt tcggtgtagg tcgttcgctc 10080
caagctgggc tgtgtgcacg aaccccccgt tcagcccgac cgctgcgcct tatccggtaa 10140
ctatcgtctt gagtccaacc cggtaagaca cgacttatcg ccactggcag cagccactgg 10200
taacaggatt agcagagcga ggtatgtagg cggtgctaca gagttcttga agtggtggcc 10260
taactacggc tacactagaa ggacagtatt tggtatctgc gctctgctga agccagttac 10320

```
cttcggaaaa agagttggta gctcttgatc cggcaaacaa accaccgctg gtagcggtgg  10380
tttttttgtt tgcaagcagc agattacgcg cagaaaaaaa ggatctcaag aagatccttt  10440
gatcttttct acggggtctg acgctcagtg gaacgaaaac tcacgttaag ggattttggt  10500
catgagatta tcaaaaagga tcttcaccta gatcctttta aattaaaaat gaagttttaa  10560
atcaatctaa agtatatatg agtaaacttg gtctgacagt taccaatcga ttggtcggtc  10620
atttcgaacc ccagagtccc gctcagaaga actcgtcaag aaggcgatag aaggcgatgc  10680
gctgcgaatc gggagcggcg ataccgtaaa gcacgaggaa gcggtcagcc cattcgccgc  10740
caagctcttc agcaatatca cgggtagcca acgctatgtc ctgatagcgg tccgccacac  10800
ccagccggcc acagtcgatg aatccagaaa agcggccatt ttccaccatg atattcggca  10860
agcaggcatc gccatgggtc acgacgagat cctcgccgtc gggcatgcgc gccttgagcc  10920
tggcgaacag ttcggctggc gcgagcccct gatgctcttc gtccagatca tcctgatcga  10980
caagaccggc ttccatccga gtacgtgctc gctcgatgcg atgtttcgct tggtggtcga  11040
atgggcaggt agccggatca agcgtatgca gccgccgcat tgcatcagcc atgatggata  11100
ctttctcggc aggagcaagg tgagatgaca ggagatcctg ccccggcact tcgcccaata  11160
gcagccagtc ccttcccgct tcagtgacaa cgtcgagcac agctgcgcaa ggaacgcccg  11220
tcgtggccag ccacgatagc cgcgctgcct cgtcctgcag ttcattcagg gcaccggaca  11280
ggtcggtctt gacaaaaaga accgggcgcc cctgcgctga cagccggaac acggcggcat  11340
cagagcagcc gattgtctgt tgtgcccagt catagccgaa tagcctctcc acccaagcgg  11400
ccggagaacc tgcgtgcaat ccatcttgtt caatcatgcg aaacgatcct catcctgtct  11460
cttgatcaga tcttgatccc ctgcgccatc agatccttgg cggcaagaaa gccatccagt  11520
ttactttgca gggcttccc                                               11539
```

<210> 6
<211> 36
<212> DNA
<213> Artificial

<220>
<223> Primer

<400> 6
acgcgtcgac ctgtaacgac aacaaaacga gggtag 36

<210> 7
<211> 30
<212> DNA
<213> Artificial

<220>
<223> Primer

<400> 7
acgcgtcgac ctgcgacagt gacagacctg 30

<210> 8
<211> 12348
<212> DNA
<213> Artificial

<220>
<223> Vector

<400> 8

atcgattgga tgcccgaggc atagactgta caaaaaaaca gtcataacaa gccatgaaaa 60
ccgccactgc gccgttacca ccgctgcgtt cggtcaaggt tctggaccag ttgcgtgagc 120
gcatacgcta cttgcattac agtttacgaa ccgaacaggc ttatgtcaat tcgcctctca 180
ggcgccgctg gtgccgctgg ttggacgcca agggtgaatc cgcctcgata ccctgattac 240
tcgcttcctg cgccctctca ggcggcgata ggggactggt aaaacgggga ttgcccagac 300
gcctcccccg ccccttcagg ggcacaaatg cggccccaac ggggccacgt agtggtgcgt 360
tttttgcgtt tccacccttt tcttcctttt ccctttttaaa ccttttagga cgtctacagg 420
ccacgtaatc cgtggcctgt agagtttaaa aagggacgga tttgttgcca ttaagggacg 480
gatttgttgt taagaaggga cggatttgtt gttgtaaagg gacggatttg ttgtattgtg 540
ggacgcagat acagtgtccc cttatacaca aggaatgtcg aacgtggcct cacccccaat 600
ggtttacaaa agcaatgccc tggtcgaggc cgcgtatcgc ctcagtgttc aggaacagcg 660
gatcgttctg gcctgtatta gccaggtgaa gaggagcgag cctgtcaccg atgaagtgat 720
gtattcagtg acggcggagg acatagcgac gatggcgggt gtccctatcg aatcttccta 780
caaccagctc aaagaagcgg ccctgcgcct gaaacggcgg gaagtccggt taacccaaga 840
gcccaatggc aaggggaaaa gaccgagtgt gatgattacc ggctgggtgc aaacaatcat 900
ctaccgggag ggtgagggcc gtgtagaact caggttcacc aaagacatgc tgccgtacct 960
gacggaactc accaaacagt tcaccaaata cgccttggct gacgtggcca agatggacag 1020
cacccacgcg atcaggcttt acgagctgct catgcaatgg gacagcatcg gccagcgcga 1080
aatagaaatt gaccagctgc gaaagtggtt tcaactggaa ggccggtatc cctcgatcaa 1140
ggacttcaag ttgcgagtgc ttgatccagc cgtgacgcag atcaacgagc acagcccgct 1200
acaggtggag tgggcgcagc gaaagaccgg gcgcaaggtc acacatctgt tgttcagttt 1260
tggaccgaag aagcccgcca aggcggtggg taaggcccca gcgaagcgca aggccgggaa 1320
gatttcagat gctgagatcg cgaaacaggc tcgccctggt gagacatggg aagcggcccg 1380
cgctcgacta acccagatgc cgctggatct ggcctagagg ccgtggccac cacggcccgg 1440
cctgcctttc aggctgcatt attgaagcat ttatcagggt tattgtctca tgagcggata 1500
catatttgaa tgtatttaga aaaataaaca aaagagtttg tagaaacgca aaaaggccat 1560
ccgtcaggat ggccttctgc ttaatttgat gcctggcagt ttatggcggg cgtcctgccc 1620

gccaccctcc gggccgttgc ttcgcaacgt tcaaatccgc tcccggcgga tttgtcctac 1680
tcaggagagc gttcaccgac aaacaacaga taaaacgaaa ggcccagtct ttcgactgag 1740
cctttcgttt tatttgatgc ctggcagttc cctactctcg catggggaga ccccacacta 1800
ccatcggcgc tacggcgttt cacttctgag ttcggcatgg ggtcaggtgg gaccaccgcg 1860
ctactgccgc caggcaaatt ctgttttatc agaccgcttc tgcgttctga tttaatctgt 1920
atcaggctga aaatcttctc tcatccgcca aaacagaagc ttggctgcag gtcgacctgc 1980
gacagtgaca gacctgatta cttagaaaac atatgacgca ccaagactga gaatgaaagg 2040
atcaacctca attttagtag aaacaggaac cgggccaaga gtacctgtga cgtccgtttt 2100
gaaaggaata taacgcacat ctgaatttag catccatgag ttaccaaggt catatctaag 2160
gccaacctga aaagcaggcg cccatttatc cttaatatca aacgaactca aagcaccgtc 2220
ggttttatca aaaaatagca cccgaccaac accaacccca acatatggat aaagtcgctc 2280
aaagctatcg taatgatatt gaagcgaaag aattgcaggg ccgtaatcaa cttcactgac 2340
tcttcccagc gaggagattg atttctcacc ttgaaattta gccctagctg gcaccccaac 2400
aaaaaaatcc accgctatat ttgagctaac aaaataggcg atatcaaacg taagtgttgt 2460
atcattacca atacttacat cagcatttgg caaagccccc cctccaacat ttagatcgcc 2520
aagctcctca cccacataga ccttagaaaa attgaagcta gcgacccagt caccctgatt 2580
atagccagca gatttcgccg gataattttc attcgcccat gcagtggccg cccccaaaac 2640
aaaattagca accaacaccg gcatcgcgat tactttataa ttagaaaaac tcattgtgct 2700
accctcgttt tgttgtcgtt acaggtcgac aagcgctgaa tgggtatcgg cactagagtt 2760
taacttggct aggctaattg gtatggtcat tttatttttg tcctgaatta cctagaatga 2820
cttgaagttt taatcttcac ttttcctcgt agagcacata gtcttctttc gtagccccgc 2880
aatccggaca gcaccagtca tcaggaatat cctcaaagcg agtacctgga gtaaaaccct 2940
cggcctcatc gcccaacgcc tcatcatata tatggccaca agtaatacat atccacttca 3000
agtatgcttt cccgccttgg gcatcagacc ttggaggttg agttttatat agatcttggc 3060
ctttaacgtc ggcactaggc aatttctcta agcttgtcgg cgcaaccact gcttcagcag 3120
ttaaacttgt gccactaacc tcggataaat ttggcgaagt atgggttgag gtgacgccct 3180
tttcacctac gccgctctca attaacatga agtcaagctt gtctcgaacg gcgcaatcgg 3240
ggcagcacca atcctcagga ataaggtgcc aaggcgtacc tggagaaaac ccctcatgca 3300
cattacccgc actctcatca taaacataat tacaatccgg gcatttatag ctagccatac 3360
tcatcaccat gtcaaattgt tattttgcgt tgcggcaaat ttgcgttatt tatttctcat 3420
cttcttcctt gtggaagatt ttcttacaac ctgtggggtg ctcgcctgat caagaacttc 3480
agtcttagcc gcacagccct catttttatt ttcttgatta agtgggatca ctaattctgc 3540

ggccaaaact gccattccag ttggagtagc atctgaaata ccactttccg agttggcaag 3600
ctgactattc acccctagct gtgtttcttt cgagggagaa tccgcgagaa agataaagtc 3660
caccttgtct cgaactgcgc agtccgggca tgcccaatct ttggggatat cattccagct 3720
ggtgttcggg tggaaacctt cgtgcggctc ccccttattt tcatcataga tatactgaca 3780
atctggacac tggtaccttg acattctccc actctcctat taactcagcg ttagtcgctg 3840
ctccgacaac ttcatcagag tgtaactatc ggagcaggtc gccttcaaag caattacaga 3900
gaggcaatca accctcagca ctttagcgaa gtgcactctt tgtgagagct ttcaacgccg 3960
cacataaaat tgaagcactt attgtaaata tcgaccgccg ggctctgcgt gctcacataa 4020
ctacgatgct accgcagagg tactcgaact atgaccagca ctactagtgc caaatttttt 4080
caaataggtt tctcgcatcg aatcatcaat ttggatctta ttaaggtcac caccagccca 4140
atctactacc ttgggatcca taactgatct aaaccactga ggaatcatcg ccatcaaaaa 4200
tgcaccaggg taacccgtcg gaagagccgg caggccggga aaatcccgaa gtgactgata 4260
agaacgtgtt ggatgcgcgt ggtgatccga gtgccgctga aggtggaaca gcactagatt 4320
agagacgatg tgattactat tccaagaatg gtgcggcttt tgatgctcat atcgaccgtc 4380
ctccattttt tgacggagca agccgtaatg ttcaatatag ttcgcactgg tcagctgcca 4440
ccaaccgaaa gccatttgaa tcggcaggaa caccagcatc ttaggtccaa acaaggcaag 4500
gagaacggcg taaagaataa ctgtgatgat cattggttgg aggatttcat tatcgaaact 4560
ccaaacgctt tggccacggc gcgaaaggcg ttgttcctca agcccccaag cacgaataaa 4620
tgctcctggg atctcacgga ttgaaaactt ataaatgctt tctcccatcc gggatgttgc 4680
aggatccatc ggtgtagcga catcacggtg atgaccctta ttatgctcaa taaagaagtg 4740
accgtaccct acgacagcca acacaatttt ggccatccaa cgatcaaaag tctccttctt 4800
gtgaccgagt tcgtgtcctg tattgagcgc tagtccgttc acgataccca gtgacaaggc 4860
aagcgcacca atttcaagcc aagacattgg ctgagttccg acccaccatg ctgacacaat 4920
taatgcagcg taatgcatag gaactgttag atatgtcaaa actcgatagt accgctcctt 4980
ctctagtttc ggcaccactt cttcaggcgg attattaaag tcctcaccaa acatcgcatc 5040
aagcaatgga agtgcgccgt accatacgag caataccagc ccataaaaaa tcccccaacc 5100
agtttcattt gcaagccaga ttccgatcat cggagtagcc ggccacaaag ttgatagtat 5160
ccagagatat ttctttttat ctacgtactc tggagcggaa tccagaactc tgtgtttctc 5220
aagcatatgg aattctccaa tttttattaa attagtcgct acgagattta agacgtaatt 5280
ttatgcctaa ctgagaaagt taagccgccc actctcactc tcgacatctt aaacctgagc 5340
taatcggacg cttgcgccaa ctacacctac gggtagtttt tgctccgtcg tctgctggaa 5400
aaacacgagc tggccgcaag catgccaggt accgcgagct actcgcgacg gctgaaagca 5460
ccgaaatgag cgagctatct ggtcgatttt gacccggtgc ccgtcttcaa aatcggcgaa 5520

ggccgaagtc ggccagaaat agcggcctac ttcagacctt ccctagtaaa tattttgcac 5580
caccgatcat gccgactaca cttaagtgta gttttaatat ttaacaccgt aacctatggt 5640
gaaaatttcc agtcagctgg cgcgagaata gcataatgaa aataataata aataatgatt 5700
tcccggtcgc taaggtcgga gcggatcaaa ttacgactct agtaagtgcc aaagttcata 5760
gttgcatata tcggccaaga ttgagtatcg cggatggagc cgctcccaga gtatgccttt 5820
acagagcccc acctggatat gggaaaaccg ttgctcttgc gttcgagtgg ctacgccaca 5880
gaacagccgg acgtcctgca gtgtggcttt ctttaagagc cagttcttac agtgaatttg 5940
atatctgcgc agagattatt gagcagcttg aaactttcga aatggtaaaa ttcagccgtg 6000
tgagagaggg tgtgagcaag cctgcgctct tgcgagacct tgcatctagt ctttggcaga 6060
gcacctcgaa taacgagata gaaacgctag tttgtttgga taatattaat catgacttag 6120
acttgccgtt gttgcacgca cttatggagt ttatgttaaa tacaccaaaa aatatcaggt 6180
ttgcagttgc aggcaataca ataaaagggt tctcgcagct taaacttgca ggcgctatgc 6240
gggagtacac cgagaaagac ttggccttta gcgcagaaga ggcggtggcg ttagcggagg 6300
cagagtctgt tcttggagtt cctgaagaac agatagagac cttggtgcaa gaagttgagg 6360
ggtggcctgc tcttgtagtt tttttgttaa agcgtgagtt gccggccaag catatttcag 6420
cagtagttga agtagacaat tactttaggg atgaaatatt tgaggcgatt cccgagcgct 6480
atcgtgtttt tcttgcaaat tcttcattgc tcgatttcgt gacgcctgat caatacaatt 6540
atgtattcaa atgcgtcaat ggggtctcat gtattaagta tttaagcact aattacatgt 6600
tgcttcgcca tgtgagcggt gagccagcgc agtttacact gcatccagta ctgcgtaatt 6660
ttctacgaga aattacttgg actgaaaatc ctgctaaaag atcctacctg cttaagcgtg 6720
cagctttctg gcattggcgt agaggtgaat accagtatgc aatacgaata tccctacggg 6780
cgaatgactg tcgctgggca gtcagcatgt ctgagagaat aattttagat ttgtcatttc 6840
gtcagggcga aatagatgcg ctgagacagt ggctgttaga gctgccgaag caggcctggc 6900
accaaaaacc catagtgctt attagttacg cgtgggtatt gtatttcagt cagcaaggcg 6960
cgcgagcaga gaagttaatt aaagacctat cttcacaatc cgataaaaaa aataaatggc 7020
aagaaaagga atggctgcag cttgtgcttg caataggtaa agcaaccaaa gatgaaatgc 7080
tttcgagtga ggagctctgt aataagtgga ttagtttatt tggggattca aacgcagttg 7140
gaaaaggggc cgcgctaacc tgtttggctt ttatttttgc cagtgagtat agatttgcag 7200
agttggagaa ggtgctggct caggcccaag ccgtgaataa atttgcaaaa caaaattttg 7260
cttttggttg gctgtatgtc gcgaggtttc aacaagccct agcaagcgga aaaatgggct 7320
gggcgaggca gattataact caagcacgca cagacagtcg cgcgcagatg atggaatccg 7380
agtttacttc gaaaatgttt gacgctctag agcttgagtt acattatgaa ttgcgctgct 7440

tggacacctc agaagaaaag ctctccaaaa ttttagagtt catttccaat cacggggtga 7500

cagacgtgtt tttttccgta tgccgtgctg tgtcagcttg gcggcttgga aggagtgacc 7560

taaatggctc cattgagata ttggagtggg cgaaggcgca tgcggttgaa aaaaatctac 7620

caagattgga agttatgagc caaattgaga tctatcagcg cttagtctgt caaggcataa 7680

cgggcataaa taatttaaaa actcttgaag atcataagat tttctccgga cagcactcag 7740

ccccccctaaa agcacgcctg ctgcttgttc aatcactagt gctttcccga gatcggaact 7800

ttcatagtgc cgcgcacaga gcgttattgg ctattcagca agcccgtaaa attaacgcgg 7860

gccagctgga agtccgtgga ttattgtgtt tggccggagc gcaggcaggt gccggtgatt 7920

taaaaaaggc tcagcttaac attgtttatg cagtggagat agcaaaacag cttcaatgct 7980

ttcaaacagt tcttgatgaa gtatgtttaa ttgagcgaat aataccggct tcatgtgaag 8040

ccttcacagc agttaattta gatcaagcga ttggggcttt tagtcttccg cgaatagttg 8100

agattggaaa gtccgcagag aataaagctg acgctttatt gacacggaag cagattgctg 8160

tcttgaggct tgtaaaagag ggtgctcaa acaaacaaat agcaacaaat atgcatgtca 8220

ccgaagatgc tataaagtgg catatgagga aaatatttgc caccttgaat gtagtgaatc 8280

gcacgcaagc aacaattgaa gctgagcgtc aaggaattat ctaaaataat cggcattaag 8340

tgatatagtg aaaagtatac cggagagaga attatggcaa tcgttgttgt tggcgctggt 8400

acagctggag taaatgctgc gttctggctt cgtcaatatg gttataaagg ggaaattagg 8460

atttttagca gggagtctgt ggcgccttat cagcggcctc ctctatccaa ggcttttctg 8520

acaagtgaga ttgcagaatc cgcagtgcca ttaaagccag aaggtttttta tacgaataac 8580

aatattacca tttcgttaaa tacaccgatt gtatcaatcg acgtggggcg taagatagtt 8640

tcttctaaag atggaaaaga atacgcgtat gaaaaattga ttcttgcaac acctgctagc 8700

gcacgtaggt taacctgcga ggggtctgaa ctgtctgggg tctgctattt acgcagtatg 8760

gaagacgcca aaaatttacg taggaaactt gtggagagtg cgtctgttgt tgtgttgggc 8820

ggcggagtaa tcgggcttga agtcgcctca gctgcggtgg gcttagggaa gagggtcaca 8880

gtgatagaag ccaccccgcg tgtaatggcg cgcgtggtta cgccggcagc agcaaactta 8940

gtcagagccc gcctggaggc tgaaggaatt gagttcaagc tgaatgcgaa attaacgtct 9000

ataaagggca ggaatggcca tgttgaacaa tgcgtacttg aaagtggaga agaaattcag 9060

gcggatctga ttgtagttgg aatcggtgct atcccagagc tagagctggc aactgaggcg 9120

gcccttgaag tgagtaatgg tgttgtggtc gatgatcaga tgtgtacatc ggatacaagt 9180

atatatgcaa tcggcgactg cgcaatggct agaaatcctt tttggggaac gatggtacgt 9240

ttagagacaa ttcataatgc ggttacacac gctcaaattg tcgcaagtag catctgtggc 9300

acatcaacac cagcaccaac cccaccacgg ttctggtctg atcttaaagg gatggcgctg 9360

caaggacttg gtgctctaaa ggactacgat aaactcgttg ttgcaattaa taacgaaact 9420

cttgaactag aagtccttgc gtacaagcag gagcgactga ttgcaactga gacaataaat 9480

ttgcctaaac gtcaaggtgc gcttgcaggg agtataaaat tacctgatta gcaatgatgc 9540

tcagccactc gaaccaacgg tcgcgatagg gacggcagtt acctgccgcc ccccgcactc 9600

cgtacgtgcg gaactaccgc gtaaaatgtg gcccaggctg ttatgtggcg cttgggcggg 9660

gaagtattgc catatttggt gatgaccgtt ttctacgcca cataaatcgg tggtggctat 9720

ggtgggattt cccttgctga aatgggagat ccgatcatgt tcgagctctt attcaaatac 9780

actgctgtgt tggcggtaag cgttctcgag ctcatagtcc acgacgcccg tgattttgta 9840

gccctggccg acggccagca ggtaggccga caggctcatg ccggccgccg ccgccttttc 9900

ctcaatcgct cttcgttcgt ctggaaggca gtacaccttg ataggtgggc tgcccttcct 9960

ggttggcttg gtttcatcag ccatccgctt gccctcatct gttacgccgg cggtagccgg 10020

ccagcctcgc agagcaggat tcccgttgag caccgccagg tgcgaataag ggacagtgaa 10080

gaaggaacac ccgctcgcgg gtgggcctac ttcacctatc ctgcccggct gacgccgttg 10140

gatacaccaa ggaaagtcta cacgaaccct ttggcaaaat cctgtatatc gtgcgaaaaa 10200

ggatggatat accgaaaaaa tcgctataat gaccccgaag cagggttatg cagcggaaaa 10260

gcgctgcttc cctgctgttt tgtggaatat ctaccgactg gaaacaggca aatgcaggaa 10320

attactgaac tgaggggaca ggcgagagag gatcaatggc tatctggggg accgagggct 10380

gtcgctgcgc caaggcacga ttggagatcc cctatgcggt gtgaaatacc gcacagatgc 10440

gtaaggagaa aataccgcat caggcgctct tccgcttcct cgctcactga ctcgctgcgc 10500

tcggtcgttc ggctgcggcg agcggtatca gctcactcaa aggcggtaat acggttatcc 10560

acagaatcag gggataacgc aggaaagaac atgtgagcaa aaggccagca aaaggccagg 10620

aaccgtaaaa aggccgcgtt gctggcgttt ttccataggc tccgcccccc tgacgagcat 10680

cacaaaaatc gacgctcaag tcagaggtgg cgaaacccga caggactata aagataccag 10740

gcgtttcccc ctggaagctc cctcgtgcgc tctcctgttc cgaccctgcc gcttaccgga 10800

tacctgtccg cctttctccc ttcgggaagc gtggcgcttt ctcatagctc acgctgtagg 10860

tatctcagtt cggtgtaggt cgttcgctcc aagctgggct gtgtgcacga accccccgtt 10920

cagcccgacc gctgcgcctt atccggtaac tatcgtcttg agtccaaccc ggtaagacac 10980

gacttatcgc cactggcagc agccactggt aacaggatta gcagagcgag gtatgtaggc 11040

ggtgctacag agttcttgaa gtggtggcct aactacggct acactagaag gacagtattt 11100

ggtatctgcg ctctgctgaa gccagttacc ttcggaaaaa gagttggtag ctcttgatcc 11160

ggcaaacaaa ccaccgctgg tagcggtggt ttttttgttt gcaagcagca gattacgcgc 11220

agaaaaaaag gatctcaaga agatcctttg atcttttcta cggggtctga cgctcagtgg 11280

aacgaaaact cacgttaagg gattttggtc atgagattat caaaaaggat cttcacctag 11340

```
atccttttaa attaaaaatg aagttttaaa tcaatctaaa gtatatatga gtaaacttgg  11400
tctgacagtt accaatcgat tggtcggtca tttcgaaccc cagagtcccg ctcagaagaa  11460
ctcgtcaaga aggcgataga aggcgatgcg ctgcgaatcg ggagcggcga taccgtaaag  11520
cacgaggaag cggtcagccc attcgccgcc aagctcttca gcaatatcac gggtagccaa  11580
cgctatgtcc tgatagcggt ccgccacacc cagccggcca cagtcgatga atccagaaaa  11640
gcggccattt tccaccatga tattcggcaa gcaggcatcg ccatgggtca cgacgagatc  11700
ctcgccgtcg ggcatgcgcg ccttgagcct ggcgaacagt tcggctggcg cgagcccctg  11760
atgctcttcg tccagatcat cctgatcgac aagaccggct tccatccgag tacgtgctcg  11820
ctcgatgcga tgtttcgctt ggtggtcgaa tgggcaggta gccggatcaa gcgtatgcag  11880
ccgccgcatt gcatcagcca tgatggatac tttctcggca ggagcaaggt gagatgacag  11940
gagatcctgc cccggcactt cgcccaatag cagccagtcc cttcccgctt cagtgacaac  12000
gtcgagcaca gctgcgcaag gaacgcccgt cgtggccagc cacgatagcc gcgctgcctc  12060
gtcctgcagt tcattcaggg caccggacag gtcggtcttg acaaaaagaa ccgggcgccc  12120
ctgcgctgac agccggaaca cggcggcatc agagcagccg attgtctgtt gtgcccagtc  12180
atagccgaat agcctctcca cccaagcggc cggagaacct gcgtgcaatc catcttgttc  12240
aatcatgcga aacgatcctc atcctgtctc ttgatcagat cttgatcccc tgcgccatca  12300
gatccttggc ggcaagaaag ccatccagtt tactttgcag ggcttccc             12348
```